Europäisches Patentamt

European Patent Office

Office européen des brevets

(19)

(11) EP 1 272 218 B1

(12) **EUROPEAN PATENT SPECIFICATION**

(45) Date of publication and mention
of the grant of the patent:
**23.03.2005 Bulletin 2005/12**

(51) Int Cl.⁷: **A61K 45/06**, A61P 29/00

(21) Application number: **01910097.3**

(22) Date of filing: **16.03.2001**

(86) International application number:
**PCT/IB2001/000391**

(87) International publication number:
**WO 2001/076576 (18.10.2001 Gazette 2001/42)**

(54) **A PHARMACEUTICAL COMPOSITION FOR TREATMENT OF ACUTE, CHRONIC PAIN AND/OR NEUROPATHIC PAIN AND MIGRAINES**

PHARMAZEUTISCHE ZUSAMMENSETZUNG ZUR BEHANDLUNG VON AKUTEM SCHMERZ, CHRONISCHEM SCHMERZ UND/ODER NEUROPATHISCHEM SCHMERZ UND MIGRÄNE

COMPOSITION PHARMACEUTIQUE SERVANT A TRAITER UNE DOULEUR CHRONIQUE AIGUE ET/OU UNE DOULEUR NEUROPHATIQUE OU DES MIGRAINES

(84) Designated Contracting States:
**AT BE CH CY DE DK ES FI FR GB GR IE IT LI LU MC NL PT SE TR**
Designated Extension States:
**AL LT LV MK RO SI**

(30) Priority: **07.04.2000 US 195738 P**

(43) Date of publication of application:
**08.01.2003 Bulletin 2003/02**

(73) Proprietor: **Pfizer Products Inc.**
**Groton, Connecticut 06340 (US)**

(72) Inventors:
• **COE, Jotham, Wadsworth**
**Groton, CT 06340 (US)**
• **HARRIGAN, Edmund, Patrick**
**Groton, CT 06340 (US)**
• **O'NEILL, Brian, Thomas**
**Groton, CT 06340 (US)**
• **SANDS, Steven, Bradley**
**Groton, CT 06340 (US)**
• **WATSKY, Eric, Jacob**
**Groton, CT 06340 (US)**

(74) Representative: **Ruddock, Keith Stephen**
**Pfizer Limited,**
**UK Patent Department,**
**Ramsgate Road**
**Sandwich, Kent CT13 9NJ (GB)**

(56) References cited:
**EP-A- 0 955 301**          **EP-A- 1 078 637**
**WO-A-98/18798**          **WO-A-99/55680**

• **DECKER M W ET AL: "THERAPEUTIC POTENTIAL OF NEURONAL NICOTINIC ACETYLCHOLINE RECEPTOR AGONISTS AS NOVEL ANALGESICS" BIOCHEMICAL PHARMACOLOGY, PERGAMON, OXFORD, GB, vol. 58, 15 September 1999 (1999-09-15), pages 917-923, XP000983313 ISSN: 0006-2952**
• **BOIDO, C.C.; SPARATORE, F.: "Synthesis and preliminary pharmacological evaluation of some cytisine derivatives" IL FARMACO, vol. 54, no. 7, 30 July 1999 (1999-07-30), pages 438-451, XP002190877**

**Description**

Background of the Invention

The present invention relates to pharmaceutical compositions for the treatment of acute, chronic and/or neuropathic pain and migraine in a mammal (e.g. human) comprising a nicotine receptor partial agonist (NRPA) and an analgesic agent which is an NMDA antagonist. The term NRPA refers to all chemical compounds which bind at neuronal nicotinic acetylcholine specific receptor sites in mammalian tissue and elicit a partial agonist response. A partial agonist response is defined here to mean a partial, or incomplete functional effect in a given functional assay. Additionally, a partial agonist will also exhibit some degree of antagonist activity by its ability to block the action of a full agonist (Feldman, R.S., Meyer, J.S. & Quenzer, L.F. Principles of Neuropsychopharmacology, 1997; Sinauer Assoc. Inc.). The present invention may be used to treat mammals (e.g. humans) for acute, chronic and/or neuropathic pain with a decrease in the severity of unwanted side effects such as causing nausea and/or stomach upset.

Fused azapolycylic compounds that bind to neuronal nicotinic acetylcholine specific receptor sites and are useful in modulating cholinergic function and are referred to in WO 9818798-A1, WO 9935131-A1 and WO 9955680-A1.

Analgesic agents decrease pain perception. In animal models of pain states, the above compounds inhibit acute pain perception. These compounds also inhibit pain sensitization processes in which the perception of the painfulness of a given stimulus is increased without any change in stimulus intensity. In humans, analgesic agents have also been found to decrease both acute pain perception and sensitization. Opioid analgesic agents, in particular, remain the most effective means of alleviating severe pain across a broad spectrum, including inflammatory as well as neuropathic pain states. However, even though analgesic agents have therapeutic utility in the treatment of pain, there are significant liabilities to the use of analgesic compounds. Specifically, many of these compounds that have been tested in humans can cause potentially serious side effects such as gastrointestinal complications including nausea, emesis, ulcers, and constipation, respiratory depression, and psychological and physical dependence.

Summary of Invention

The present invention relates to a pharmaceutical composition for the treatment of acute, chronic and/or neuropathic pain and migraine comprising (a) a nicotine receptor partial agonist or a pharmaceutically acceptable salt thereof; (b) an NMDA antagonist and (c) a pharmaceutically acceptable carrier; wherein the active agents "a" and "b" above are present in amounts that render the composition effective in treating acute, chronic and/or neuropathic pain, and migraine.

A nicotinic partial agonist combined with an NMDA antagonist may inhibit pain sensitization and pain perception while reducing the incidence of undesirable side effects. A nicotinic partial agonist combined with an NMDA antagonist may inhibit pain sensitization and pain perception while reducing the incidence of undesirable side effects. Nicotine has long been appreciated to have antinociceptive properties, but its use has been limited by a poor spectrum of activity, side effects, and less efficacy than opioids. This may be due to a lack of specificity of nicotine for neuromuscular, ganglionic, and central nervous system receptors. The development of nicotine partial agonists with specific receptor subtype affinities is an approach to potentially reduce side effects and enhance efficacy.

In a more specific embodiment of this invention, the nicotine receptor partial agonist is selected from:

9-bromo-1,2,3,4,5,6-hexahydro-1,5-methano-pyrido[1,2-a][1,5]diazocin-8-one;
9-chloro-1,2,3,4,5,6-hexahydro-1,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-ethyl-1,2,3,4,5,6-hexahydro-1,5-methano-pyrido[1,2-a][1,5]diazocin-8-one;
9-methyl-1,2,3,4,5,6-hexahydro-1,5-methano-pyrido[1,2-a][1,5]diazocin-8-one;
9-phenyl-1,2,3,4,5,6-hexahydro-1,5-methano-pyrido[1,2-a][1,5]diazocin-8-one;
9-vinyl-1,2,3,4,5,6-hexahydro-1,5-methano-pyrido[1,2-a][1,5]diazocin-8-one;
9-bromo-3-methyl-1,2,3,4,5,6-hexahydro-1,5-methano-pyrido[1,2-a][1,5]diazocin-8-one;
3-benzyl-9-bromo-1,2,3,4,5,6-hexahydro-1,5-methano-pyrido[1,2-a][ 1,5]diazocin-8-one;
3-benzyl-9-chloro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;
9-ethynyl-1,2,3,4,5,6-hexahydro-1,5-methano-pyrido[1,2a][1,5]diazocin-8-one;
9-(2-propenyl)-1,2,3,4,5,6-hexahydro-1,5-methano-pyrido[1,2a][1,5]diazocin-8-one;
9-(2-propyl)-1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;
9-carboxyaldehyde-1,2,3,4,5,6-hexahydro-1,5-methano-pyrido[1,2a][1,5]diazocin-8-one;
9-(2,6-difluorophenyl)-1,2,3,4,5,6-hexahydro-1,5-methano-pyrido[1,2a][1,5]diazocin-8-one;

9-phenyl-1,2,3,4,5,6-hexahydro-1,5-methano-pyrido[1,2a][1,5]diazocin-8-one;

9-(2-fluorophenyl)-1,2,3,4,5,6-hexahydro-1,5-methano-pyrido[1,2a][1,5]diazocin-8-one;

9-(4-fluorophenyl)-1,2,3,4,5,6-hexahydro-1,5-methano-pyrido[1,2a][1,5]diazocin-8-one;

9-(3-fluorophenyl)-1,2,3,4,5,6-hexahydro-1,5-methano-pyrido[1,2a][1,5]diazocin-8-one;

9-(3,5-difluorophenyl)-1,2,3,4,5,6-hexahydro-1,5-methano-pyrido[1,2a][1,5]diazocin-8-one;

9-(2,4-difluorophenyl)-1,2,3,4,5,6-hexahydro-1,5-methano-pyrido[1,2a][1,5]diazocin-8-one;

9-(2,5-difluoraphenyl)-1,2,3,4,5,6-hexahydro-1,5-methano-pyrido[1,2a][1,5]diazocin-8-one;

6-methyl-5-oxo-6,13-diazatetracyclo[9.3.1.0$^{2,10}$.0$^{4,8}$]pentadeca-2(10),3,8-triene;

5-oxo-6,13-diazatetracyclo[9.3.1.0$^{2,10}$.0$^{4,8}$]pentadeca-2(10),3,8-triene;

6-oxo-5,7,13-triazatetracyclo[9.3.1.0$^{2,10}$.0$^{4,8}$]pentadeca-2(10),3,8-triene;

4,5-difluoro-10-aza-tricyclo[6.3.1.0$^{2,7}$]dodeca-2(7),3,5-triene;

5-fluoro-10-aza-tricyclo[6.3.1.0$^{2,7}$]dodeca-2(7),3,5-triene-4-carbonitrile;

4-ethynyl-5-fluoro-10-aza-tricyclo[6.3.1.0$^{2,7}$]dodeca-2(7),3,5-triene;

5-ethynyl-10-aza-tricyclo[6.3.1.0$^{2,7}$]dodeca-2(7),3,5-triene-4-carbonitrile;

6-methyl-5-thia-5-dioxa-6,13-diazatetracyclo[9.3.1.0$^{2,10}$.0$^{4,8}$]pentadeca-2(10),3,8-triene;

10-aza-tricyclo[6.3.1.0$^{2,7}$]dodeca-2(7),3,5-triene;

4-fluoro-10-aza-tricyclo[6.3.1.0$^{2,7}$]dodeca-2(7),3,5-triene;

4-methyl-10-aza-tricyclo[6-3.1.0$^{2,7}$]dodeca-2(7),3,5-triene;

4-trifluoromethyl-10-aza-tricyclo[6.3.1.0$^{2,7}$]dodeca-2(7),3,5-triene;

4-nitro-10-azatricyclo[6.3.1.0$^{2,7}$]dodeca-2(7),3,5-triene;

7-methyl-5,7,13-triazatetracyclo[9.3.1.0$^{2,10}$.0$^{4,8}$]pentadeca-2(10),3,5,8-tetraene;

6-methyl-5,7,13-triazatetracyclo[9.3.1.0$^{2,10}$.0$^{4,8}$]pentadeca-2(10),3,5,8-tetraene;

6,7-dimethyl-5,7,13-triazatetracyclo[9.3.1.0$^{2,10}$.0$^{4,8}$]pentadeca-2(10),3,5,8-tetraene;

6-methyl-7-phenyl-5,7,13-triazatetracyclo[9.3.1.0$^{2,10}$.0$^{4,8}$]pentadeca-2(10),3,5,8-tetraene;

6,7-dimethyl-5,8,14-triazatetracyclo[10.3.1.0$^{2,11}$.0$^{4,9}$]hexadeca-2(11 ),3,5,7,9-pentaene;

5,8,14-triazatetracyclo[10.3.1.0$^{2,11}$.0$^{4,9}$]hexadeca-2(11),3,5,7,9-pentaene;

14-methyl-5,8,14-triazatetracyclo[10.3.1.0$^{2,11}$.0$^{4,9}$]hexadeca-2(11),3,5,7,9-pentaene;

5-oxa-7,13-diazatetracyclo[9.3.1.0$^{2,10}$.0$^{4,8}$]pentadeca-2(10),3,6,8-tetraene;

6-methyl-5-oxa-7,13-diazatetracyclo[9.3.1.0$^{2,10}$.0$^{4,8}$]pentadeca-2(10),3,6,8-tetraene;

4-chloro-10-azatricyclo[6.3.1.0$^{2,7}$]dodeca-2(7),3,5-triene;

10-azatricyclo[6.3.1.0$^{2,7}$]dodeca-2(7),3,5-trien-4-yl cyanide;

1-(10-azatricyclo[6.3.1.0$^{2,7}$]dodeca-2(7),3,5-trien-4-yl)-1-ethanone;

10-azatricyclo[6.3.1.0$^{2,7}$]dodeca-2(7),3,5-trien-4-ol;

7-methyl-5-oxa-6,13-diazatetracyclo[9.3.1.0$^{2,10}$.0$^{4,8}$]pentadeca-2,4(8),6,9-tetraene;

4,5-dichloro-10-azatricyclo[6.3.1.0$^{2,7}$]dodeca-2(7),3,5-triene;

11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7),3,5-triene-5-carbonitrile:

5-fluoro-11-azatricyclo[7.3.1.0$^{2,7}$]trideca-2(7),3,5-triene-4-carbonitrile;

6-methyl-7-thia-5,14-diazatetracyclo[10.3.1.0$^{2,10}$.0$^{4,8}$]hexadeca-2(10),3,5,8-tetraene;

6-methyl-5,7,14-triazatetracyclo[10.3.1.0$^{2,10}$.0$^{4,8}$]hexadeca-2(10),3,5,8-tetraene;

6,7-dimethyl-5,7,14-triazatetracyclo[10.3.1.0$^{2,10}$.0$^{4,8}$]hexadeca-2(10),3,5,8-tetraene;

5,7,14-triazatetracyclo[10.3.1.0$^{2,10}$.0$^{4,8}$]hexadeca-2(10),3,5,8-tetraene;

5,6-dimethyl-5,7,14-triazatetracyclo[10.3.1.0$^{2,10}$.0$^{4,8}$]hexadeca-2(10),3,6,8-tetraene;

5-methyl-5,7,14-triazatetracyclo[10.3.1.0$^{2,10}$.0$^{4,8}$]hexadeca-2(10),3,6,8-tetraene;

6-(trifluoromethyl)-7-thia-5,14-diazatetracyclo[10.3.1.0$^{2,10}$.0$^{4,8}$]hexadeca-2(10),3,5,8-tetraene;

5,8,15-triazatetracyclo[11.3.1.0$^{2,11}$.0$^{4,9}$]heptadeca-2(11),3,5,7,9-pentaene;

7-methyl-5,8,15-triazatetracyclo[11.3.1.0$^{2,11}$.0$^{4,9}$]heptadeca-2(11),3,5,7,9-pentaene;

6-methyl-5,8,15-triazatetracyclo[11.3.1.0$^{2,11}$.0$^{4,9}$]heptadeca-2(11),3,5,7,9-pentaene;

6,7-dimethyl-5,8,15-triazatetracyclo[11.3.1.0$^{2,11}$.0$^{4,9}$]heptadeca-2(11),3,5,7,9-pentaene;

7-oxa-5,14-diazatetracyclo[10.3.1.0$^{2,10}$.0$^{4,8}$]hexadeca-2(10),3,5,8-tetraene;

6-methyl-7-oxa-5,14-diazatetracyclo[10.3.1.0$^{2,10}$.0$^{4,8}$]hexadeca-2(10),3,5,8-tetraene;

5-methyl-7-oxa-6,14-diazatetracyclo[10.3.1.0$^{2,10}$.0$^{4,8}$]hexadeca-2(10),3,5,8-tetraene;

6-methyl-5-oxa-7,14-diazatetracyclo[10.3.1.0$^{2,10}$.0$^{4,8}$]hexadeca-2(10),3,6,8-tetraene;

7-methyl-5-oxa-6,14-diazatetracyclo[10.3.1.0$^{2,10}$.0$^{4,8}$]hexadeca-2(10),3,6,8-tetraene;

4,5-difluoro-11-azatricyclo[7.3.1.0$^{2,7}$]trideca-2(7),3,5-triene;

4-chloro-5-fluoro-11-azatricyclo[7.3.1.0$^{2,7}$]trideca-2(7),3,5-triene;

5-chloro-4-fluoro-11-azatricyclo[7.3.1.0$^{2,7}$]trideca-2(7),3,5-triene;
4-(1-ethynyl)-5-fluoro-11-azatricyclo[7.3.1.0$^{2,7}$]trideca-2(7),3,5-triene;
5-(1-ethynyl)-4-fluoro-11-azatricyclo[7.3.1.0$^{2,7}$]trideca-2(7),3,5-triene;
5,6-difluoro-11-aza-tricyclo[7.3.1.0$^{2,7}$]trideca-2,4,6-triene;
6-tritluoromethyl-11-aza-tricyclo[7.3.1.0$^{2,7}$]trideca-2,4,6-triene;
6-methoxy-11-aza-tricyclo[7.3.1.0$^{2,7}$]trideca-2(7),3,5-triene;
11-aza-tricyclo[7.3.1.0$^{2,7}$]trideca-2(7),3,5-trion-6-ol;
6-fluoro-11-aza-tricyclo[7.3.1.0$^{2,7}$]trideca-2(7),3,5-triene;
11-aza-tricyclo[7.3.1.0$^{2,7}$]trideca-2(7),3,5-trien-5-ol;
4-nitro-11-aza-tricyclo[7.3.1.0$^{2,7}$]trideca-2(7),3,5-triene;
5-nitro-11-aza-tricyclo[7.3.1.0$^{2,7}$]trideca-2(7),3,5-triene;
5-fluoro-11-aza-tricyclo[7.3.1.0$^{2,7}$]trideca-2(7),3,5-triene; and
6-hydroxy-5-methoxy-11-aza-tricyclo[7.3.1.0$^{2,7}$]trideca-2(7),3,5-triene and
their pharmaceutically acceptable salts and their optical isomers.

Preferably, the nicotine receptor partial agonist is selected from

9-bromo-1,2,3,4,5,6-hexahydro-1,5-methano-pyrido[1,2-a][1,5]diazocin-8-one;
9-chloro-1,2,3,4,5,6-hexahydro-1,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;
9-carboxyaldehyde-1,2,3,5,6-hexahydro-1,5-methano-pyrido[1,2a][1,5]diazocin-8-one;
9-(2,6-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2-a][1,5]diazocin-8-one;
9-(2-fluorophenyl)-1,2,3,4,5,6-hexahydro-1,5-methano-pyrido[1,2a][1,5]diazocin-8-one;
6-methyl-5-thia-5-dioxa-6,13-diazatetracyclo[9.3.1.0$^{2,10}$.0$^{4,8}$]pentadeca-2(10),3,8-triene;
4-fluoro-10-aza-tricyclo[6.3.1.0$^{2,7}$]dodeca-2(7),3,5-triene;
4-trifluoromethyl-10-aza-tricyclo[6.3.1.0$^{2,7}$]dodeca-2(7),3,5-triene;
4-nitro-10-azatricyclo[6.3.1.0$^{2,7}$]dodeca-2(7),3,5-triene;
6-methyl-5,7,13-triazatetracyclo[9.3.1.0$^{2,10}$.0$^{4,8}$]pentadeca-2(10),3,5,8-tetraene;
6,7-dimethyl-5,8,14-triazatetracyclo[10.3.1.0$^{2,11}$.0$^{4,9}$]hexadeca-2(11),3,5,7,9-pentaene;
5,8,14-triazatetracyclo[10.3.1.0$^{2,11}$.0$^{4,9}$]hexadeca-2(11),3,5,7,9-pentaene;
5-oxa-7,13-diazatetracyclo[9.3.1.0$^{2,10}$.0$^{4,8}$]pentadeca-2(10),3,6,8-tetraene;
6-methyl-5-oxa-7,13-diazatetracyclo[9.3.1.0$^{2,10}$.0$^{4,8}$]pentadeca-2(10),3,6,8-tetraene;
10-azatricyclo[6.3.1.0$^{2,7}$]dodeca-2(7),3,5-trien-4-yl cyanide;
1-(10-azatricyclo[6.3.1.0$^{2,7}$]dodeca-2(7), 3,5-trien-4-yl)-1-ethanone;
11-azatricyclo[7.3.1.0$^{2,7}$]trideca-2(7),3,5-triene-5-carbonitrile;
1-[11-azatricyclo[7.3.1.0$^{2,7}$]trideca-2(7),3,5-trien-5-yl]-1-ethanone;
1-[11-azatricyclo[7.3.1.0$^{2,7}$]trideca-2(7),3,5-trien-5-yl]-1-propanone;
4-fluoro-11-azatricyclo[7.3.1.0$^{2,7}$]trideca-2(7),3,5-triene-5-carbonitrile;
5-fluoro-11-azatricyclo[7.3.1.0$^{2,7}$]trideca-2(7),3,5-triene-4-carbonitrile;
6-methyl-7-thia-5,14-diazatetracyclo[10.3.1.0$^{2,10}$.0$^{4,8}$]hexadeca-2(10),3,5,8-tetraene;
6-methyl-5,7,14-triazatetracyclo[10.3.1.0$^{2,10}$.0$^{4,8}$]hexadeca-2(10),3,5,8-tetraene;
6,7-dimethyl-5,7,14-triazatetracyclo[10.3.1.0$^{2,10}$.0$^{4,8}$]hexadeca-2(10),3,5,8-tetraene;
6-methyl-7-oxa-5,14-diazatetracyclo[10.3.1.0$^{2,10}$.0$^{4,8}$]hexadeca-2(10),3,5,8-tetraene;
6-methyl-5-oxa-7,14-diazatetracyclo[10.3.1.0$^{2,10}$.0$^{4,8}$]hexadeca-2(10),3,6,8-tetraene;
5,6-difluoro-11-aza-tricyclo[7.3.1.0$^{2,7}$]trideca-2,4,6-triene;
6-trifluoromethyl-11-aza-tricyclo[7.3.1.0$^{2,7}$]trideca-2,4,6-triene;
6-methoxy-11-aza-tricyclo[7.3.1.0$^{2,7}$]trideca-2(7),3,5-triene;
6-fluoro-11-aza-tricyclo[7.3.1.0$^{2,7}$]trideca-2(7),3,5-triene; and
11-aza-tricyclo[7.3.1.0$^{2,7}$]trideca-2(7),3,5-trien-5-ol; and
their pharmaceutically acceptable salts and their optical isomers.

In a more specific embodiment of the invention, the NMDA antagonist is selected from dextromethorphan, 2-piperidinol-1-alkanol derivatives as described in the United States Patent No. 5,272,160 and incorporated herein by reference, eliprodil, and ifenprodil;

The invention also relates to the use of a pharmaceutical composition comprising: (a) a nicotine receptor partial agonist or a pharmaceutically acceptable salt thereof; (b) an NMDA antagonist or pharmaceutically acceptable salt thereof and (c) a pharmaceutically acceptable carrier, for the manufacture of a medicament for the treatment of acute, chronic and/or neuropathic pain and migraine in a mammal.

In another more specific embodiment of this invention the nicotine receptor partial agonist is selected from

9-bromo-1,2,3,4,5,6-hexahydro-1,5-methano-pyrido[1,2-a][1,5]diazocin-8-one;
9-chloro-1,2,3,4,5,6-hexahydro-1,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-ethyt-1,2,3,4,5,6-hexahydro-1,5-methano-pyrido[1,2-a][1,5]diazocin-8-one;
9-methyl-1,2,3,4,5,6-hexahydro-1,5-methano-pyrido[1,2-a][1,5]diazocin-8-one;
9-phenyl-1,2,3,4,5,6-hexahydro-1,5-methano-pyrido[1,2-a][1,5]diazocin-8-one;
9-vinyl-1,2,3,4,5,6-hexahydro-1,5-methano-pyrido[1,2-a][1,5]diazocin-8-one;
9-bromo-3-methyl-1,2,3,4,5,6-hexahydro-1,5-methano-pyrido[1,2-a][1,5]diazocin-8-one;
3-benzyl-9-bromo-1,2,3,4,5,6-hexahydro-1,5-methano-pyrido[1,2-a][1,5]diazocin-8-one;
3-benzyl-9-chloro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydra-1,5-methano-pyrido[1,2a][1,5]diazocin-8-one;
9-ethynyl-1,2,3,4,5,6-hexahydro-1,5-methano-pyrido[1,2a][1,5]diazocin-8-one;
9-(2-propenyl)-1,2,3,4,5,6-hexahydro-1,5-methano-pyrido[1,2a][1.5]diazocin-8-one;
9-(2-propyl)-1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;
9-carboxyaldehyde-1,2,3,4,5,6-hexahydro-1,5-methano-pyrido[1,2a][1,5]díazocin-8-one;
9-(2,6-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
9-(4-fluorophenyl)-1,2,3,4,5,6-hexahydro-1,5-methano-pyrido[1,2a][1,5]diazocin-8-one;
9-(3-fluorophenyl)-1,2,3,4,5,6-hexahydro-1,5-methano-pyrido[1,2a][1,5]diazocin-8-one;
9-(3,5-difluorophenyl)-1,2,3,4,5,6-hexahydro-1,5-methano-pyrido[1,2a][1,5]diazocin-8-one;
9-(2,4-difluorophenyl)-1,2,3,4,5,6-hexahydro-1,5-methano-pyrido[1,2a][1,5]diazocin-8-one;
9-(2,5-difluorophenyl)-1,2,3,4,5,6-hexahydro-1,5-methano-pyrido[1,2a][1,5]diazocin-8-one;
6-methyl-5-oxo-6,13-diazatetracyclo[9.3.1.0^{2,10}.0^{4,8}]pentadeca-2(10),3,8-triene;
5-oxo-6,13-diazatetracyclo[9.3.1.0^{2,10}.0^{4,8}]pentadeca-2(10),3,8-triene;
6-oxo-5,7,13-triazatetracyclo[9.3.1.0^{2,10}.0^{4,8}]pentadeca-2(10),3,8-triene;
4,5-difluoro-10-aza-tricyclo[6.3.1.0^{2,7}]dodeca-2(7),3,5-triene;
5-fluoro-10-aza-tricyclo[6.3.1.0^{2,7}]dodeca-2(7),3,5-triene-4-carbonitrile;
4-ethynyl-5-fluoro-10-aza-tricyclo[6.3.1.0^{2,7}]dodeca-2(7),3,5-triene;
5-ethynyl-10-aza-tricyclo[6.3.1.0^{2,7}]dodeca-2(7),3,5-triene-4-carbonitrile;
6-methyl-5-thia-5-dioxa-6,13-diazatetracyclo[9.3.1.0^{2,10}.0^{4,8}]pentadeca-2(10),3,8-triene;
10-aza-tricyclo[6.3.1.0^{2,7}]dodeca-2(7),3,5-triene;
4-fluoro-10-aza-tricyclo[6.3.1.0^{2,7}]dodeca-2(7),3,5-triene;
4-methyl-10-aza-tricyclo[6.3.1.0^{2,7}]dodeca-2(7),3,5-triene;
4-trifluoromethyl-10-aza-tricyclo[6.3.1.0^{2,7}]dodeca-2(7),3,5-triene;
4-nitro-10-azatricyclo[6.3.1.0^{2,7}]dodeca-2(7),3,5-triene;
7-methyl-5,7,13-triazatetracyclo[9.3.1.0^{2,10}.0^{4,8}]pentadeca-2(10),3,5,8-tetraene;
6-methyl-5,7,13-triazatetracyclo[9.3.1.0^{2,10}.0^{4,8}]pentadeca-2(10),3,5,8-tetraene;
6,7-dimethyl-5,7,13-triazatetracyclo[9.3.1.0^{2,10}.0^{4,8}]pentadeca-2(10),3,5,8-tetraene;
6-methyl-7-phenyl-5,7,13-triazatetracyclo[9.3.1.0^{2,10}.0^{4,8}]pentadeca-2(10),3,5,8-tetraene;
6,7-dimethyl-5,8,14-triazatetracyclo[10.3.1.0^{2,11}.0^{4,9}]hexadeca-2(11),3,5,7,9-pentaene;
5,8,14-triazatetracyclo[10.3.1.0^{2,11}.0^{4,9}]hexadeca-2(11),3,5,7,9-pentaene;
14-methyl-5,8,14-triazatetracyclo[10.3.1.0^{2,11}.0^{4,9}]hexadeca-2(11),3,5,7,9-pentaene;
5-oxa-7,13-diazatetracyclo[9.3.1.0^{2,10}.0^{4,8}]pentadeca-2(10),3,6,8-tetraene;
6-methyl-5-oxa-7,13-diazatetracyclo[9.3.1.0^{2,10}.0^{4,8}]pentadeca-2(10),3,6,8-tetraene;
4-chloro-10-azatricyclo[6.3.1.0^{2,7}]dodeca-2(7),3,5-triene;
10-azatricyclo[6.3.1.0^{2,7}]dodeca-2(7),3,5-trien-4-yl cyanide;
1-(10-azatricyclo[6.3.1.0^{2,7}]dodeca-2(7),3,5-trien-4-yl)-1-ethanone;
10-azatricyclo[6.3.1.0^{2,7}]dodeca-2(7),3,5-trien-4-ol;
7-methyl-5-oxa-6,13-diazatetracyclo[9.3.1.0^{2,10}.0^{4,8}]pentadeca-2,4(8),6,9-tetraene;
4,5-dichloro-10-azatricyclo[6.3.1.0^{2,7}]dodeca-2(7),3,5-triene;

11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7),3,5-triene-5-carbonitrile;

5-fluoro-11-azatricyclo[7.3.1.0$^{2,7}$]trideca-2(7),3,5-triene-4-carbonitrile;

6-methyl-7-thia-5,14-diazatetracyclo[10.3.1.0$^{2,10}$.0$^{4,8}$]hexadeca-2(10),3,5,8-tetraene;

6-methyl-5,7,14-triazatetracyclo[10.3.1.0$^{2,7}$.0$^{4,8}$]hexadeca-2(10),3,5,8-tetraene;

6,7-dimethyl-5,7,14-triazatetracyclo[10.3.1.0$^{2,10}$.0$^{4,8}$]hexadeca-2(10),3,5,8-tetraene;

5,7,14-triazatetracyclo[10.3.1.0$^{2,10}$.0$^{4,8}$]hexadeca-2(10),3,5,8-tetraene;

5,6-dimethyl-5,7,14-triazatetracyclo[10.3.1.0$^{2,10}$.0$^{4,8}$]hexadeca-2(10),3,6,8-tetraene;

5-methyl-5,7,14-triazatetracyclo[10.3.1.0$^{2,10}$.0$^{4,8}$]hexadeca-2(10),3,6,8-tetraene;

6-(trifluoromethyl)-7-thia-5,14-diazatetracyclo[10.3.1.0$^{2,10}$.0$^{4,8}$]hexadeca-2(10),3,5,8-tetraene;

5,8,15-triazatetracyclo[11.3.1.0$^{2,11}$.0$^{4,9}$]heptadeca-2(11),3,5,7,9-pentaene;

7-methyl-5,8,15-triazatetracyclo[11.3.1.0$^{2,11}$.0$^{4,9}$]heptadeca-2(11),3,5,7,9-pentaene;

6-methyl-5,8,15-triazatetracyclo[11.3.1.0$^{2,11}$.0$^{4,9}$]heptadeca-2(11),3,5,7,9-pentaene;

6,7-dimethyl-5,8,15-triazatetracyclo[11.3.1.0$^{2,11}$.0$^{4,9}$]heptadeca-2(11),3,5,7,9-pentaene;

7-oxa-5,14-diazatetracyclo[10.3.1.0$^{2,10}$.0$^{4,8}$]hexadeca-2(10),3,5,8-tetraene;

6-methyl-7-oxa-5,14-diazatetracyclo[10.3.1.0$^{2,10}$.0$^{4,8}$]hexadeca-2(10),3,5,8-tetraene;

5-methyl-7-oxa-6,14-diazatetracyclo[10.3.1.0$^{2,10}$.0$^{4,8}$]hexadeca-2(10),3,5,8-tetraene;

6-methyl-5-oxa-7,14-diazatetracyclo[10.3.1.0$^{2,10}$.0$^{4,8}$]hexadeca-2(10),3,6,8-tetraene;

7-methyl-5-oxa-6,14-diazatetracyclo[10.3.1.0$^{2,10}$.0$^{4,8}$]hexadeca-2(10),3,6,8-tetraene;

4,5-difluoro-11-azatricyclo[7.3.1.0$^{2,7}$]trideca-2(7),3,5-triene;

4-chloro-5-fluoro-11-azatricyclo[7.3.1.0$^{2,7}$]trideca-2(7),3,5-triene;

5-chloro-4-fluoro-11-azatricyclo[7.3.1.0$^{2,7}$]trideca-2(7),3,5-triene;

4-(1-ethynyl)-5-fluoro-11-azatricyclo[7.3.1.0$^{2,7}$]trideca-2(7),3,5-triene;

5-(1-ethynyl)-4-fluoro-11-azatricyclo[7.3.1.0$^{2,7}$]trideca-2(7),3,5-triene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7),3,5-triene;

11-aza-tricyclo[7.3.1.0$^{2,7}$]trideca-2(7),3,5-trien-6-ol;

6-fluoro-11-aza-tricyclo[7.3.0$^{2,7}$]trideca-2(7),3,5-triene;

11-aza-tricyclo[7.3.1.0$^{2,7}$]trideca-2(7),3,5-trien-5-ol;

4-nitro-11-aza-tricyclo[7.3.1.0.$^{2,7}$]trideca-2(7),3,5-triene;

5-nitro-11-aza-tricyclo[7.3.1.0$^{2,7}$]trideca-2(7),3,5-triene;

5-fluoro-11-aza-tricyclo[7.3.1.0$^{2,7}$]trideca-2(7),3,5-triene; and

6-hydroxy-5-methoxy-11-aza-tricyclo[7.3.1.0$^{2,7}$]trideca-2(7),3,5-triene and their pharmaceutically acceptable salts and their optical isomers.

Preferably, the nicotine receptor partial agonist is selected from

9-bromo-1,2,3,4,5,6-hexahydro-1,5-methano-pyrido[1,2-a][1,5]diazocin-8-one;

9-chloro-1,2,3,4,5,6-hexahydro-1,5-methano-pyrido[1,2-a][1,5]diazocin-8-one;

9-fluoro-1,2,3,4,5,6-hexahydro-1,5-methano-pyrido[1,2-a][1,5]diazocin-8-one;

9-acetyl-1,2,3,4,5,6-hexahydro-1,5-methano-pyrido[1,2a][1,5]diazocin-8-one;

9-iodo-1,2,3,4,5,6-hexahydro-1,5-methano-pyrido[1,2a][1,5]diazocin-8-one;

9-cyano-1,2,3,4,5,6-hexahydro-1,5-methano-pyrido[1,2a][1,5]diazocin-8-one;

9-carbomethoxy-1,2,3,4,5,6-hexahydro-1,5-methano-pyrido[1,2a][1,5]diazocin-8-one;

9-carboxyaldehyde-1,2,3,4,5,6-hexahydro-1,5-methano-pyrido[1,2a][1,5]diazocin-8-one;

9-(2,6-difluorophenyl)-1,2,3,4,5,6-hexahydro-1,5-methano-pyrido[1,2a][1,5]diazocin-8-one;

9-phenyl-1,2,3,4,5,6-hexahydro-1,5-methano-pyrido[1,2a][1,5]diazocin-8-one;

9-(2-fluorophenyl)-1,2,3,4,5,6-hexahydro-1,5-methano-pyrido[1,2a][1,5]diazocin-8-one;

6-methyl-5-thia-5-dioxa-6,13-diazatetracyclo[9.3.1.0$^{2,10}$.0$^{4,8}$pentadeca-2(10),3,8-triene;

4-fluoro-10-aza-tricyclo[6.3.1.0$^{2,7}$]dodeca-2(7),3,5-triene;

4-trifluoromethyl-10-aza-tricyclo[6.3.1.0$^{2,7}$]dodeca-2(7),3,5-triene;

4-nitro-10-azatricyclo[6.3.1.0$^{2,7}$]dodeca-2(7),3,5-triene;

6-methyl-5.7,13-triazatetracyclo[9.3.1.0$^{2,10}$.0$^{4,8}$]pentadeca-2(10),3,5,8-tetraene;

6,7-dimethyl-5,8,14-triazatetracyclo[10,3.1.0.$^{2,11}$.0$^{4,9}$]hexadeca-2(11),3,5,7,9-pentaene;

5,8,14-triazatetracyclo[10.3.1.0$^{2,11}$.0$^{4,9}$]hexadeca-2(11),3,5,7,9-pentaene;

5-oxa-7,13-diazatetracyclo[9.3.1.0$^{2,10}$.0$^{4,8}$]pentadeca-2(10),3,6,8-tetraene;

6-methyl-5-oxa-7,13-diazatetracyclo[9,3,1,0$^{2,10}$.0$^{4,8}$]pentadeca-2(10),3,6,8-tetraene;

10-azatricyclo[6.3.1.0$^{2,7}$]dodeca-2(7),3,5-trien-4-yl cyanide;

1-(10-azatricyclo(6.3.1.0$^{2,7}$]dodeca-2(7),3,5-trien-4-yl)-1-ethanone;

11-azatricyclo[7.3.1.0$^{2,7}$]trideca-2(7),3,5-triene-5-carbonitrile;

1-[11-azatricyclo[7.3.1.0$^{2,7}$-]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7),3,5-triene-5-carbonitrile;

5-fluoro-11-azatricyclo[7.3.1.0$^{2,7}$]trideca-2(7),3,5-triene-4-carbonitrile;

6-methyl-7-thia-5,14-diazatetracyclo[10.3.1.0$^{2,10}$.0$^{4,8}$]hexadeca-2(10),3,5,8-tetraene;

6-methyl-5,7,14-triazatetracyclo(10.3.1.0$^{2,10}$.0$^{4,8}$]hexadeca-2(10),3,5,8-tetraene;

6,7-dimethyl-5,7,14-triazatetracyclo[10.3.1.0$^{2,10}$.0$^{4,8}$]hexadeca-2(10),3,5,8-tetraene;

6-methyl-7-oxa-5,14-diazatetracyclo[10.3.1.0$^{2,10}$.0$^{4,8}$]hexadeca-2(10),3,5,8-tetraene;

6-methyl-5-oxa-7,14-diazatetracyclo[10.3.1.0$^{2,10}$.0$^{4,8}$]hexadeca-2(10),3,6,8-tetraene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7),3,5-triene;

6-fluoro-11-aza-tricyclo[7.3.1.0$^{2,7}$]trideca-2(7),3,5-triene; and

11-aza-tricyclo[7.3.1.0$^{2,7}$]tridica-2(7),3,5-trien-5-ol; and the pharmaceutically acceptable salts and optical isomers of the foregoing compounds.

In a more specific embodiment the NMDA antagonists are selected from dextromethorphan, 2-piperidinol-1-alkanol derivatives as described in the United States Patent No. 5,272,160, eliprodil, ifenprodil.

This invention also relates to a pharmaceutical composition for treating a disorder or condition selected from the group consisting of diseases and conditions in which pain predominates, including acute pain, chronic pain, neuropathic pain and migraine, and including soft tissue and peripheral damage, such as acute trauma, osteoarthritis, rheumatoid arthritis, musculo- skeletal pain, particularly after trauma, spinal pain, dental pain, myofascial pain syndromes, headache, episiotomy pain, and bums; deep and visceral pain, such as heart pain, muscle pain, eye pain, orofacial pain, for example, odontalgia, abdominal pain, gynaecological pain, for example, dysmenorrhea, and labor pain; pain associated with nerve and root damage, such as pain associated with peripheral nerve disorders, for example, nerve entrapment and brachial plexus avulsions, amputation, peripheral neuropathies, tic douloureux, atypical facial pain, nerve root damage, and arachnoiditis; pain associated with carcinoma, often referred to as cancer pain; central nervous system pain, such as pain due to spinal cord or brain stem damage; low back pain; sciatica; headache, including migraine, acute or chronic tension headache, cluster headache, temporomandibular pain and maxillary sinus pain; ankylosing spondylitis, gout; post operative pain; and scar pain, in a mammal, including a human, comprising: (a) a nicotine receptor partial agonist or a pharmaceutically acceptable salt thereof; (b) an NMDA antagonist or a pharmaceutically acceptable salt thereof and (c) a pharmaceutically acceptable carrier, wherein the active agents "a" and "b" above are present in amounts that render the composition effective in treating acute, chronic and/or neuropathic pain and migraine.

This invention also relates to the use of a pharmaceutical composition comprising: (a) a nicotine receptor partial agonist or a pharmaceutically acceptable salt thereof; (b) an NMDA antagonist or a pharmaceutically acceptable salt thereof and (c) a pharmaceutically acceptable carrier, for the manufacture of a medicament for treating a disorder or condition selected from the group consisting of diseases and conditions in which pain predominates, including acute pain, chronic pain, neuropathic pain and migraine, and including soft tissue and peripheral damage, such as acute trauma, osteoarthritis, rheumatoid arthritis, musculoskeletal pain, particularly after trauma, spinal pain, dental pain, myofascial pain syndromes, headache, episiotomy pain, and burns; deep and visceral pain, such as heart pain, muscle pain, eye pain, orofacial pain, for example, odontalgia, abdominal pain, gynaecological pain, for example, dysmenorrhea, and labor pain; pain associated with nerve and root damage, such as pain associated with peripheral nerve disorders, for example, nerve entrapment and brachial plexus avulsions, amputation, peripheral neuropathies, tic douloureux, atypical facial pain, nerve root damage, and arachnoiditis; pain associated with carcinoma, often referred to as cancer pain; central nervous system pain, such as pain due to spinal cord or brain stem damage; low back pain; sciatica; headache, including migraine, acute or chronic tension headache, cluster headache, temporomandibular pain and maxillary sinus pain; ankylosing spondylitis, gout; post operative pain; and scar pain, in a mammal, including a human.

The term "treating" as used herein, refers to reversing, alleviating, inhibiting or slowing the progress of, or preventing the disorder or condition to which such term applies, or one or more symptoms of such disorder or condition. The term "treatment", as used herein, refers to the act of treating, as "treating" is defined immediately above.

The chemist of ordinary skill will recognize that certain compounds of this invention will contain one or more atoms which may be in a particular stereochemical or geometric configuration, giving rise to stereoisomers and configurational isomers. All such isomers and mixture thereof are included in this invention. Hydrates of the compounds of this invention are also included.

The chemist of ordinary skill will recognize that certain combinations of heteroatom-containing substituents listed in this invention define compounds which will be less stable under physiological conditions (e.g., those containing acetal or animal linkages). According, such compounds are less preferred.

Detailed Description of the Invention

In combination with the NRPA, the invention includes an NMDA antagonist. The particular NRPA compounds listed above, which can be employed in the method and pharmaceutical, compositions of this invention, can be made by processes known in the chemical arts, for example by the methods described in WO 9818798 A1, WO 9935131-A1 and United States Provisional Patent Application No. 60/083,556 filed April 29, 1998. Some of the preparation methods useful for making the compounds of this invention may require protection of remote functionality (i.e., primary amine, secondary amine, carboxyl). The need for such protection will vary depending on the nature of the remote functionality and the conditions of the preparation methods. The need for such protection is readily determined by one skilled in the art, and is described in examples carefully described in the above cited applications. The starting materials and reagents for the NRPA compounds employed in this invention are also readily available or can be easily synthesized by those skilled in the art using conventional methods of organic synthesis. Some of the compounds used herein are related to, or are derived from compounds found in nature and accordingly many such compounds are commercially available or are reported in the literature or are easily prepared from other commonly available substances by methods which are reported in the literature.

Some of the NRPA compounds employed in this invention are ionizable at physiological conditions. Thus, for example some of the compounds of this invention are acidic and they form a salt with a pharmaceutically acceptable cation. All such salts are within the scope of this invention and they can be prepared by conventional methods. For example, they can be prepared simply by contacting the acidic and basic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate.

In addition, some of the NRPA compounds employed in this invention are basic, and they form a salt with a pharmaceutically acceptable anion. All such salts are within the scope of this invention and they can be prepared by conventional methods. For example, they can be prepared simply by contacting the acidic and basic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate.

In addition, when the NRPA compounds employed in this invention form hydrates or solvates they are also within the scope of the invention.

Some of the compounds of this invention are chiral, and as such are subject to preparation via chiral synthetic routes, or separable by conventional resolution or chromatographic means. All optical forms of the compounds of this invention are within the scope of the invention.

The utility of the NRPA compounds employed in the present invention as medicinal agents in the treatment of pain in mammals (e.g. humans) is demonstrated by the activity of the compounds of this invention in conventional assays and, in particular the assays described below. These include neuronal nicotinic receptor binding and animal models of pain. Such assays also provide a means whereby the activities of the compounds of this invention can be compared between themselves and with the activities of other known compounds. The results of these comparisons are useful for determining dosage levels in mammals, including humans, for the treatment of such diseases.

Administration of the compositions of this invention can be via any method which delivers a compound of this invention systemically and/or locally. These methods include oral routes and transdermal routes, etc. Generally, the compounds of this invention are administered orally, but parenteral administration may be utilized (e.g., intravenous, intramuscular, subcutaneous or intramedullary). The two different compounds of this invention can be co-administered simultaneously or sequentially in any order, or a single pharmaceutical composition comprising a NRPA as described above and an NMDA antagonist as described above in a pharmaceutically acceptable carrier can be administered.

The amount and timing of compounds administered will, of course, be based on the judgement of the prescribing physician. Thus, because of patient to patient variability, the dosages given below are a guideline and the physician may titrate doses of the agent to achieve the activity that the physician considers appropriate for the individual patient. In considering the degree of activity desired, the physician must balance a variety of factors such as cognitive function, age of the patient, presence of preexisting disease, as well as presence of other diseases (e.g., cardiovascular). The following paragraphs provide preferred dosage ranges for the various components of this invention (based on average human weight of 70 kg).

Biological Assays

Procedures

Receptor binding assay: The effectiveness of the active compounds in suppressing nicotine binding to specific receptor sites is determined by the following procedure which is a modification of the methods of Lippiello, P. M. and Fernandes, K. G. (in The Binding of L-[3H]Nicotine To A Single Class of High-Affinity Sites in Rat Brain Membranes, Molecular Pharm., 29, 448-54, (1986)) and Anderson, D. J. and Arneric, S. P. (in Nicotinic Receptor Binding of 3H-Cystisine, 3H-Nicotine and 3H-Methylcarmbamylcholine In Rat Brain, European J. Pharm., 253, 261-67 (1994)). Male Sprague-Dawley rats (200-300 g) from Charles River were housed in groups in hanging stainless steel wire cages and were maintained on a 12 hour light/dark cycle (7 a.m.-7 p.m. light period). They received standard Purina Rat Chow and water *ad libitum.* The rats were killed by decapitation. Brains were removed immediately following decapitation. Membranes were prepared from brain tissue according to the methods of Lippiello and Fernandez (Molec Pharmacol, 29, 448-454, (1986) with some modifications. Whole brains were removed, rinsed with ice-cold buffer, and homogenized at $0°$ in 10 volumes of buffer (w/v) using a Brinkmann Polytron™, setting 6, for 30 seconds. The buffer consisted of 50 mM Tris HCl at a pH of 7.5 at room temperature. The homogenate was sedimented by centrifugation (10 minutes; 50,000 x g; $0°$ to $4°C$). The supernatant was poured off and the membranes were gently resuspended with the Polytron and centrifuged again (10 minutes; 50,000 x g; 0 to $4°C$. After the second centrifugation, the membranes were resuspended in assay buffer at a concentration of 1.0g/ 100mL. The composition of the standard assay buffer was 50 mM Tris HCl, 120 mM NaCl, 5 mM KCl, 2 mM $MgCl_2$, 2 mM $CaCl_2$ and has a pH of 7.4 at room temperature.

Routine assays were performed in borosilicate glass test tubes. The assay mixture typically consisted of 0.9 mg of membrane protein in a final incubation volume of 1.0 mL. Three sets of tubes were prepared wherein the tubes in each set contained 50μL of vehicle, blank, or test compound solution, respectively. To each tube was added 200μL of [3H]-nicotine in assay buffer followed by 750μL of the membrane suspension. The final concentration of nicotine in each tube was 0.9 nM. The final concentration of cytisine in the blank was 1μM. The vehicle consisted of deionized water containing 30μL of 1 N acetic acid per 50 mL of water. The test compounds and cytisine were dissolved in vehicle. Assays were initiated by vortexing after addition of the membrane suspension to the tube. The samples were incubated at $0°$ to $4°$ C in an iced shaking water bath. Incubations were terminated by rapid filtration under vacuum through Whatman GP/B™ glass fiber filters using a Brandel™ multi-manifold tissue harvester. Following the initial filtration of the assay mixture, filters were washed two times with ice-cold assay buffer (5 m each). The filters were then placed in counting vials and mixed vigorously with 20 ml of Ready Safe™ (Beckman) before quantification of radioactivity. Samples were counted in a LKB Wallach Rackbeta™ liquid scintillation counter at 40-50% efficiency. All determinations were in triplicate.

Calculations: Specific binding (C) to the membrane is the difference between total binding in the samples containing vehicle only and membrane (A) and non-specific binding in the samples containing the membrane and cytisine (B), i.e.,

$$\text{Specific binding} = (C) = (A) - (B).$$

Specific binding in the presence of the test compound (E) is the difference between the total binding in the presence of the test compound (D) and non-specific binding (B), i.e., (E) = (D) -(B).

$$\% \text{ Inhibition} = (1-((E)/(C))) \text{ times } 100.$$

The compounds of the invention that were tested in the above assay exhibited $IC_{50}$ values of less than 10μM.

Assay methods for acute pain:

Tail flick

Tail-flick testing, which tests reflex nociceptive function, follows the procedure derived from D'Amour and Smith (D'Amour, F.E., and Smith, E., A method for determining loss of pain sensation, J. Pharmacol. Exp. Therapeutics, 72:74-79, 1941). The test is done with a standard apparatus obtained from Columbus instruments. A beam of radiant heat from a high intensity light is focussed on the tail while the animal is manually restrained. The response time is recorded, defined as the interval between the onset of the heat stimulus and the abrupt flick of the tail. As soon as the response occurs, the heat is removed from the tail. A cutoff time of 14 seconds (or less) is set to prevent damage to the tail of an animal with deficient sensory function. The test is administered to an animal three times in a session, varying the exact location of the heat spot on the tail to minimize sensitization and potential damage. Control animals have a tail flick response latency of approximately 4.5-5.0 seconds.

Hot plate

The hot-plate test, involving central as well as peripheral mechanisms of nociceptive responding, is conducted with an IITC model 39D Analgesia Meter. A rat is placed on a surface which is maintained at 55 degrees C. The surface is surrounded by a cylinder of clear plexiglass (10 in high). The latency between the time the rat is placed on the surface and the time it licks either hindpaw or attempts escape is the hot plate latency, and the animal is immediately removed from the apparatus at this time. One determination is recorded. To prevent tissue damage, tests of non-responsive animals are terminated after 40 sec., with that time assigned as the response latency. During the week prior to testing, the rats are given brief exposures to the non-functional hot-plate to adapt them to the testing situation. Control animals respond between 10-15 seconds.

Assay method for acute and chronic pain

Formalin test

This test does not allow escape from the stimulus, but is established as a standard means to test responses to a longer-duration nociceptive chemical stimulus. The response has two phases that appear to have separate mechanisms, distinct from one another and from the responses tested using the tests listed above, that can be independently investigated only by use of this test or similar tests (see (Tjolsen et al., 1992, cited below).

Animals are adapted to the testing situation without formalin injection during the week before the test. Fifty ml of 5% formalin solution is injected subcutaneously into the dorsal surface of the right hind paw with a 30 guage needle. The rat is then placed in an open plexiglass chamber to allow unhindered observation of the formalin-injected paw. Nociception-related behavior is quantified by counting the incidence of spontaneous flinching or shaking of the injected paw. Flinches are counted for each individual animal in periods of 1 minute starting at 1-2 min. after formalin injection, then at 5-minute intervals during the interval from 10-60 minutes. After the observation period of 1 hour, animals are sacrificed. Previous studies report that the duration of the painful stimulus in the formalin test is limited, and beyond one hour the pain is minimal (for review see Tjolsen, A., Berge, O-G., Hunskaar, S., Rosland, J.H., and Hole, K., The formalin test: an evaluation of the method, Pain, 51:5-17, 1992)

Assay method for neuropathic pain

Recently, several animal models of neuropathic pain have been developed in rats.
Bennett Model: [G. J. Bennett, Pain, 33, 87-107, **1988**] Under anesthesia, the rat is placed in a prone position and an incision is made in the skin over the thigh. The fascia between the gluteus and biceps femoris muscle is dissected and the right common sciatic nerve is exposed at the level of the midthigh. Proximal to its trifurcation, the nerve is carefully dissected from its surrounding tissue over a distance of about 8 mm. In the experimental group, ligatures are loosely tied around the common sciatic nerve. A similar dissection is performed on the contralateral side, except that the nerve is not ligated (sham surgery). A group of control animals with bilateral sham surgery is also included. Comparison of the results of the experimental and control sides of the experimental rats allows the detection of possible, contralateral effects of the nerve ligation.
In general, an effective dosage for the NRPA in the range of 0.001 to 200 mg/kg/day, preferably 0.001 to 10.0 mg/kg/day.
In particular, an effective dosage for 2-piperidinol-1-alkanol derivatives as described in United States. Patent No.5, 272,160, when used in the combination compositions and methods of this invention, is in the range of 0.1 to 20 mg/kg/day.
In particular, an effective dosage for eliprodil, when used in the combination compositions and methods of this invention, is in the range of 0.01 to 0.4 mg/kg/day
In particular, an effective dosage for ifenprodil, when used in the combination compositions and methods of this invention, is in the range of 0.01 to 0.3 mg/kg/day.
The compositions of the present invention are generally administered in the form of a pharmaceutical composition comprising at least one of the compounds of this invention together with a pharmaceutically acceptable vehicle or diluent. Thus, the compounds of this invention can be administered individually or together in any conventional oral, parenteral or transdermal dosage form.
For oral administration a pharmaceutical composition can take the form of solutions, suspensions, tablets, pills, capsules, powders, and the like. Tablets containing various excipient such as sodium citrate, calcium carbonate and calcium phosphate are employed along with various disintegrants such as starch and preferably potato or tapioca starch and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the compounds of this invention can be combined with various sweetening agents, flavoring agents, coloring agents, emulsifying agents and/or suspending agents, as well as such diluents as water, ethanol, propylene glycol, glycerin and various like combinations thereof.
For purposes of parenteral administration, solutions in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes. In this connection, the sterile aqueous media employed are all readily obtainable by standard techniques well-known to those skilled in the art.

For purposes of transdermal (e.g.,topical) administration, dilute sterile, aqueous or partially aqueous solutions (usually in about 0.1% to 5% concentration), otherwise similar to the above parenteral solutions, are prepared.

Methods of preparing various pharmaceutical compositions with a certain amount of active ingredient are known, or will be apparent in light of this disclosure, to those skilled in this art. For examples, see Remington's Pharmaceutical Sciences, Mack Publishing Company, Easter, Pa., 15th Edition (1975).

Pharmaceutical compositions according to the invention may contain 0.1%-95% of the compound(s) of this invention, preferably 1%-70%. In any event, the composition or formulation to be administered will contain a quantity of a compound(s) according to the invention in an amount effective to treat the pain of the subject being treated.

**Claims**

1. The combination of a nicotine receptor partial agonist or a pharmaceutically acceptable salt thereof and an NMDA antagonist or a pharmaceutically acceptable salt thereof.

2. The combination as claimed in claim 1 wherein the NMDA antagonist is selected from a 2-piperidino-1-alkanol derivative, dextromethorphan, eliprodil and ifenprodil, their pharmaceutically active salts and their optical isomers.

3. The combination as claimed in claim 1 wherein the nicotine receptor partial agonist is selected from

9-bromo-1,2,3,4,5,6-hexahydro-1,5-methano-pyrido[1,2-a][1,5]diazocin-8-one:
9-chloro-1,2,3,4,5,6-hexahydra-1,5-methano-pyrido[1.2-a][1,5]diazocin-8-one;
9-fluoro-1,2,3,4,5,6-hexahydro-1,5-methano-pyrido[1,2-a][1,5]diazocin-8-one;
9-ethyl-1,2,3,4,5,6-hexahydro-1,5-methano-pyrido[1,2-a][1,5]diazocin-8-one;
9-methyl-1,2,3,4,5,6-hexahydro-1,5-methano-pyrido[1,2-a][1,5]diazocin-8-one;
9-phenyl-1,2,3,4,5,6-hexahydro-1,5-methano-pyrido[1,2-a][1,5]diazocin-8-one-,
9-vinyl-1,2,3,4,5,6-hexahydro-1,5-methano-pyrido[1,2-a][1,5]diazocin-8-one;
9-bromo-3-methyl-1,2,3,4,5,6-hexahydro-1,5-methano-pyrido[1,2-a][1,5]diazocin-8-one;
3-benzyl-9-bromo-1,2,3,4,5,6-hexahydro-1,5-methano-pyrido[1,2-a][1,5]diazocin-8-one;
3-benzyl-9-chloro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;
9-ethynyl-1,2,3,4,5,6-hexahydro-1,5-methano-pyrido[1,2a][1,5]diazocin-8-one:
9-(2-propenyl)-1,2,3,4,5,6-hexahydro-1,5-methano-pyrido[1,2a][1,5]diazocin-8-one;
9-(2-propyl)- 1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;
9-carboxyaldehyde-1,2,3,4,5,6-hexahydro-1,5-methano-pyrido[1,2a][1,5]diazocin-8-one;
9-(2,6-difluorophenyl)-1,2,3,4,5,6-hexahydro-1,5-methano-pyrido[1,2a][1,5]diazocin-8-one:
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
9-(4-fluorophenyl)-1,2,3,4,5,6-hexahydro-1,5-methano-pyrido[1,2a][1,5]diazocin-8-one;
9-(3-fluorophenyl)-1,2,3,4,5,6-hexahydro-1,5-methano-pyrido[1,2a][1,5]diazocin-8-one:
9-(3,5-difluorophenyl)-1,2,3,4,5,6-hexahydro-1,5-methano-pyrido[1,2a][1,5]diazocin-8-one;
9-(2,4-difluorophenyl)-1,2,3,4,5,6-hexahydro-1,5-methano-pyrido[1,2a][1,5]diazocin-8-one;
9-(2,5-difluorophenyl)-1,2,3,4,5,6-hexahydro-1,5-methano-pyrido[1,2a][1,5]diazocin-8-one;
6-methyl-5-oxo-6,13-diazatetracyclo[9.3.1.0$^{2,10}$.0$^{4,8}$]pentadeca-2(10),3,8-triene;
5-oxo-6,13-diazatetracyclo[9.3.1.0$^{2,10}$.0$^{4,8}$]pentadeca-2(10),3,8-triene;
6-oxo-5,7,13-triazatetracyclo[9.3.1.0$^{2,10}$.0$^{4,8}$]pentadeca-2(10),3,8-triene;
4,5-difluoro-10-aza-tricyclo[6.3.1.0$^{2,7}$]dodeca-2(7),3,5-triene;
5-fluoro-10-aza-tricyclo[6.3.1.0$^{2,7}$]dodeca-2(7),3,5-triene-4-carbonitrile;
4-ethynyl-5-fluoro-10-aza-tricyclo[6.3.1.0$^{2,7}$]dodeca-2(7),3,5-triene;
5-ethynyl-10-aza-tricyclo[6.3.1.0$^{2,7}$]dodeca-2(7),3,5-triene-4-carbonitrile;
6-methyl-5-thia-5-dioxa-6,13-diazatetracyclo[9.3.1.0$^{2,10}$.0$^{4,6}$]pentadeca-2(10),3,8-triene;
10-aza-tricyclo[6.3.1.0$^{2,7}$]dodeca-2(7),3,5-triene;
4-fluoro-10-aza-tricyclo[6.3.1.0$^{2,7}$]dodeca-2(7),3,5-triene;

4-methyl-10-aza-tricyclo[6.3.1.0$^{2,7}$]dodeca-2(7),3,5-triene;

4-trifluoromethyl-10-aza-tricyclo[6.3.1.0$^{2,7}$]dodeca-2(7),3,5-triene;

4-nitro-10-azatricyclo[6.3.1.0$^{2,7}$]dodeca-2(7),3,5-triene;

7-methyl-5,7,13-triazatetracyclo[9.3.1.0$^{2,10}$.0$^{4,8}$]pentadeca-2(10),3,5,8-tetraene;

6-methyl-5,7,13-triazatetracyclo[9.3.1.0$^{2,10}$.0$^{4,8}$]pentadeca-2(10),3,5,8-tetraene;

6,7-dimethyl-5,7,13-triazatetracyclo[9.3.1.0$^{2,10}$.0$^{4,8}$]pentadeca-2(10),3,5,8-tetraene;

6-methyl-7-phenyl-5,7,13-triazatetracyclo[9.3.1.0$^{2,10}$.0$^{4,6}$]pentadeca-2(10),3,5,8-tetraene;

6,7-dimethyl-5,8,4-triazatetracyclo[10.3.1.0$^{2,11}$.0$^{4,9}$]hexadeca-2(11),3,5,7,9-pentaene;

5,8,14-triazatetracyclo[10.3.1.0$^{2,11}$.0$^{4,9}$]hexadeca-2(11),3,5,7,9-pentaene;

14-methyl-5,8,14-triazatetracyclo[10.3.1.0$^{2,11}$.0$^{4,9}$]hexadeca-2(11),3,5,7,9-pentaene;

5-oxa-7,13-diazatetracyclo[9.3.1.0$^{2,10}$.0$^{4,8}$]pentadeca-2(10),3,6,8-tetraene;

6-methyl-5-oxa-7,13-diazatetracyclo[9.3.1.0$^{2,10}$.0$^{4,6}$]pentadeca-2(10),3,6,8-tetraene;

4-chloro-10-azatricyclo[6.3.1.0$^{2,7}$]dodeca-2(7),3,5-triene;

10-azatricyclo[6.3.1.0$^{2,7}$]dodeca-2(7),3,5-trien-4-yl cyanide;

1-(10-azatricyclo[6.3.1.0$^{2,7}$]dodeca-2(7),3,5-trien-4-yl)-1-ethanone;

10-azatricyclo[6.3.1.0$^{2,7}$]dodeca-2(7),3,5-trien-4-ol;

7-methyl-5-oxa-6,13-diazatetracyclo[9.3.1.0$^{2,10}$.0$^{4,8}$]pentadeca-2,4(8),6,9-tetraene;

4,5-dichloro-10-azatricyclo[6.3.1.0$^{2,7}$]dodeca-2(7),3,5-triene;

11-azatricyclo[7.3.1.0$^{2,7}$]trideca-2(7),3,5-triene-5-yl-carbonitrile;

1-[11-azatricyclo[7.3.1.0$^{2,7}$]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7),3,5-triene-5-carbonitrile;

5-fluoro-11-azatricyclo[7.3.1.0$^{2,7}$]trideca-2(7),3,5-triene-4-carbonitrile;

6-methyl-7-thia-5,14-diazatetracyclo[10.3.1.0$^{2,10}$.0$^{4,8}$]hexadeca-2(10),3,5,8-tetraene;

6-methyl-5,7,14-triazatetracyclo[10.3.1.0$^{2,10}$.0$^{4,8}$]hexadeca-2(10),3,5,8-tetraene;

6,7-dimethyl-5,7,14-triazatetracyclo

[10.3.1.0$^{2,10}$.0$^{4,6}$]hexadeca-2(10),3,5,8-tetraene;

5,7,14-triazatetracyclo[10.3.1.0$^{2,7}$.0$^{4,8}$]hexadeca-2(10),3,5,8-tetraene;

5,6-dimethyl-5,7,14-triazatetracyclo[10.3.1.0$^{2,10}$.0$^{4,8}$]hexadeca-2(10),3,6,8-tetraene;

5-methyl-5,7,14-triazatetracyclo[10.3.1.0$^{2,10}$.0$^{4,8}$]hexadeca-2(10),3,6,8-tetraene;

6-(trifluoromethyl)-7-thia-5,14-diazatetracyclo[10.3.1.0$^{2,10}$.0$^{4,8}$]hexadeca-2(10),3,5,8-tetraene;

5,8,15-triazatetracyclo[11.3.1.0$^{2,11}$.0$^{4,9}$]heptadeca-2(11),3,5,7,9-pentaene;

7-methyl-5,8,15-triazatetracyclo[11.3.1.0$^{2,11}$.0$^{4,9}$]heptadeca-2(11),3,5,7,9-pentaene;

6-methyl-5,8,15-triazatetracyclo[11.3.1.0$^{2,11}$.0$^{4,9}$]heptadeca-2(11 ),3,5,7,9-pentaene;

6,7-dimethyl-5,8,15-triazatetracyclo[11.3.1.0$^{2,11}$.0$^{4,9}$]heptadeca-2(11),3,5,7,9-pentaene;

7-oxa-5,14-diazatetracyclo[10.3.1.0$^{2,10}$.0$^{4,8}$]hexadeca-2(10),3,5,8-tetraene;

6-methyl-7-oxa-5,14-diazatetracyclo[10.3.1.0$^{2,10}$.0$^{4,8}$]hexadeca-2(10),3,5,8-tetraene;

5-methyl-7-oxa-6,14-diazatetracyclo[10.3.1.0$^{2,10}$,0$^{4,8}$]hexadeca-2(10),3,5,8-tetraene;

6-methyl-5-oxa-7,14-diazatetracyclo[10.3.1.0$^{2,10}$.0$^{4,8}$]hexadeca-2(10),3,6,8-tetraene;

7-methyl-5-oxa-6,14-diazatetracyclo[10.3.1.0$^{2,10}$.0$^{4,8}$]hexadeca-2(10),3,6,8-tetraene;

4.5-difluoro-11-azatricyclo[7.3.1.0$^{2,7}$]trideca-2(7),3,5-triene;

4-chloro-5-fluoro-11-azatricyclo[7.3.1.0$^{2,7}$]trideca-2(7),3,5-triene;

5-chloro-4-fluoro-11-azatricyclo[7.3.1.0$^{2,7}$]trideca-2(7),3,5-triene;

4-(1-ethynyl)-5-fluoro-11-aza-tricyclo[7.3.1.0$^{2,7}$]trideca-2(7),3,5-triene;

5-(1-ethynyl)-4-fluoro-11-azatricyclo[7.3.1.0$^{2,7}$]trideca-2(7),3,5-triene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7),3,5-triene;

11-aza-tricyclo[7.3.1.0$^{2,7}$]trideca-2(7),3,5-trien-6-ol;

6-fluoro-11-aza-tricyclo[7.3.1.0$^{2,7}$]trideca-2(7),3,5-triene;

11-aza-tricyclo[7.3.1.0$^{2,7}$]trideca-2(7),3,5-trien-5-ol;

4-nitro-11-aza-tricyclo[7.3.1.0$^{2,7}$]trideca-2(7),3,5-triene;
5-nitro-11-aza-tricyclo[7.3.1.0$^{2,7}$]trideca-2(7),3,5-triene;
5-fluoro-11-aza-tricyclo[7.3.1.0$^{2,7}$]trideca-2(7),3,5-triene;
6-hydroxy-5-methoxy-11-aza-tricyclo[7.3.1.0$^{2,7}$]trideca-2(7),3,5-triene; and

their pharmaceutically acceptable salts and their optical isomers.

4. A combination as claimed in claim 3 wherein the nicotine receptor partial agonist is selected from

9-bromo-1,2,3,4,5,6-hexahydro-1,5-methano-pyrido[1,2-a][1,5]diazocin-8-one;
9-chloro-1,2,3,4,5,6-hexahydro-1,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;
9-carboxyaldehyde-1.2.3.4.5.6-hexahydro-1,5-methano-pyrido[1,2a][1,5]diazocin-8-one;
9-(2,6-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
6-methyl-5-thia-5-dioxa-6,13-diazatetracyclo[9.3.1.0$^{2,10}$.0$^{4,8}$]pentadeca-2(10),3,8-triene;
4-fluoro-10-aza-tricyclo[6.3.1.0$^{2,7}$]dodeca-2(7),3,5-triene;
4-trifluoromethyl-10-aza-tricyclo[6.3.1.0$^{2,7}$]dodeca-2(7),3,5-triene;
4-nitro-10-azatricyclo[6.3.1.0$^{2,7}$]dodeca-2(7),3,5-triene;
6-methyl-5,7,13-triazatetracyclo[9.3.1.0$^{2,10}$.0$^{4,8}$]pentadeca-2(10),3,5,8-tetraene;
6,7-dimethyl-5,8,14-triazatetracyclo[10.3.1.0$^{2,11}$.0$^{4,9}$]hexadeca-2(11),3,5,7,9-pentaene;
5,8,14-triazatetracyclo[10.3.1.0$^{2,11}$.0$^{4,9}$]hexadeca-2(11),3,5,7,9-pentaene;
5-oxa-7,13-diazatetracyclo[9.3.1.0$^{2,10}$.0$^{4,8}$]pentadeca-2(10),3,6,8-tetraene:
6-methyl-5-oxa-7,13-diazatetracyclo[9.3.1.0$^{2,10}$.0$^{4,8}$]pentadeca-2(10),3,6,8-tetraene;
10-azatricyclo[6.3.1.0$^{2,7}$]dodeca-2(7),3,5-trien-4-yl cyanide;
1-(10-azatricyclo[6.3.1.0$^{2,7}$]dodeca-2(7),3,5-trien-4-yl)-1-ethanone;
11-azatricyclo[7.3.1.0$^{2,7}$]trideca-2(7),3,5-triene-5-carbonitrile;
1-(11-azatricyclo[7.3.1.0$^{2,7}$]trideca-2(7),3,5-trien-5-yl]-1-ethanone;
1-[11-azatricyclo[7.3.1.0$^{2,7}$]trideca-2(7),3,5-trien-5-yl]-1-propanone;
4-fluoro-11-azatricyclo[7.3.1.0$^{2,7}$]trideca-2(7),3,5-triene-5-carbonitrile;
5-fluoro-11-azatricyclo[7.3.1.0$^{2,7}$]trideca-2(7),3,5-triene-4-carbonitrile;
6-methyl-7-thia-5,14-diazatetracyclo[10.3.1.0$^{2,10}$.0$^{4,8}$]hexadeca-2(10).3.5.8-tetraene:
6-methyl-5,7,14-triazatetracyclo[10.3.1.0$^{2,10}$.0$^{4,8}$]hexadeca-2(10),3,5,8-tetraene;
6.7-dimethyl-5,7,14-triazatetracyclo[10.3.1.0$^{2,10}$.0$^{4,8}$)hexadeca-2(10),3,5,8-tetraene;
6-methyl-7-oxa-5,14-diazatetracyclo[10.3.1.0$^{2,10}$.0$^{4,8}$]hexadeca-2(10),3,5,8-tetraene;
6-methyl-5-oxa-7,14-diazatetracyclo[10.3.1.0$^{2,10}$.0$^{4,8}$]hexadeca-2(10),3,6,8-tetraene;
5.6-difluoro-11-aza-tricyclo[7.3.1.0$^{2,7}$]trideca-2,4,6-triene;
6-trifluoromethyl-11-aza-tricyclo[7.3.1.0$^{2,7}$]trideca-2,4,6-triene;
6-methoxy-11-aza-tricyclo[7.3.1.0$^{2,7}$]trideca-2(7),3,5-triene;
6-fluoro-11-aza-tricyclo[7.3.1.0$^{2,7}$]trideca-2(7),3,5-triene;
11-aza-tricyclo[7.3.1.0$^{2,7}$]trideca-2(7),3,5-trien-5-ol;

and the pharmaceutically acceptable salts and optical isomers thereof.

5. A pharmaceutical composition comprising a combination as claimed in any one of claims 1 to 4 and a pharmaceutically acceptable carrier.

6. A combination as claimed in any one of claims 1 to 4 for use as a medicament.

7. The use of a combination as claimed in any one of claims 1 to 4 for the manufacture of a medicament for the treatment of acute, chronic and/or neuropathic pain and migraine.

8. The use of claim 7 wherein the nicotine receptor partial agonist and the NMDA receptor antagonist are administered substantially simultaneously.

9. The use of a combination of any one of claims 1 to 4 for the manufacture of a medicament for the treatment of a disorder or condition selected from the group consisting of diseases and conditions in which pain predominates, including acute pain, chronic pain, neuropathic pain and migraine, and including soft tissue and peripheral damage, such as acute trauma, osteoarthritis, rheumatoid arthritis, musculo- skeletal pain, particularly after trauma, spinal pain, dental pain, myofascial pain syndromes, headache, episiotomy pain. and bums; deep and visceral pain. such as heart pain, muscle pain, eye pain, orofacial pain, for example, odontalgia, abdominal pain, gynaecological pain, for example, dysmenorrhea, and labor pain; pain associated with nerve and root damage. such as pain associated with peripheral nerve disorders, for example, nerve entrapment and brachial plexus avulsions, amputation, peripheral neuropathies, tic douloureux, atypical facial pain, nerve root damage, and arachnoiditis; pain associated with carcinoma, often referred to as cancer pain; central nervous system pain, such as pain due to spinal cord or brain stem damage; low back pain: sciatica; headache, including migraine, acute or chronic tension headache, cluster headache, temporomandibular pain and maxillary sinus pain; ankylosing spondylitis, gout; post operative pain; and scar pain.

**Patentansprüche**

1. Die Kombination eines Nikotinrezeptor-Partialsagonisten oder eines pharmazeutisch akzeptablen Salzes hiervon und eines NMDA-Antagonisten oder eines pharmazeutisch akzeptablen Salzes hiervon.

2. Die Kombination wie in Anspruch 1 beansprucht worin der NMDA Antagonist ausgewählt wird aus einem 2-Piperidino-1-alkanol-derivat, Dextromethorphan, Eliprodil und Ifenprodil, ihren pharmazeutisch wirksamen Salzen und ihren optischen Isomeren.

3. Die Kombination wie in Anspruch 1 beansprucht worin der Nikotinrezeptor-Partialagonist ausgewählt wird aus:

9-Bromo-1,2,3,4,5,6-hexahydro-1,5-methano-pyrido[1,2-a][1,5]diazocin-8-on,
9-Chloro-1,2,3,4,5,6-hexahydro-1,5-methano-pyrido[1,2-a][ 1,5]diazocin-8-on,
9-Fluoro-1,2,3,4,5,6-hexahydro-1,5-methano-pyrido[1,2-a][1,5]diazocin-8-on,
9-Ethyl-1,2,3,4,5,6-hexahydro-1,5-methano-pyrido[1,2-a][1,5]diazocin-8-on,
9-Methyl-1,2,3,4,5,6-hexahydro-1,5-methano-pyrido[1,2-a][1,5]diazocin-8-on,
9-Phenyl-1,2,3,4,5,6-hexahydro-1,5-methano-pyrido[1,2-a][1,5]diazocin-8-on,
9-Vinyl-1,2,3,4,5,6-hexahydro-1,5-methano-pyrido[1,2-a][1,5]diazocin-8-on,
9-Bromo-3-methyl-1,2,3,4,5,6-hexahydro-1,5-methano-pyrido[1,2-a][1,5]diazocin-8-on,
3-Benzyl-9-bromo-1,2,3,4,5,6-hexahydro-1,5-methano-pyrido[1,2-a][1,5]diazocin-8-on,
3-Benzyl-9-chloro-1,2,3,4,5,6-hexahydro-1,5-methano-pyrido[1,2-a][1,5]diazocin-8-on,
9-Acetyl-1,2,3,4,5,6-hexahydro-1,5-methano-pyrido[1,2a][1,5]diazocin-8-on,
9-Jodo-1,2,3,4,5,6-hexahydro-1,5-methano-pyrido[1,2a][1,5]diazocin-8-on,
9-Cyano-1,2,3,4,5,6-hexahydro-1,5-methano-pyrido[1,2a][1,5]diazocin-8-on,
9-Ethynyl-1,2,3,4,5,6-hexahydro-1,5-methano-pyrido[1,2a][1,5]diazocin-8-on,
9-(2-Propenyl)-1,2,3,4,5,6-hexahydro-1,5-methano-pyrido[1,2a][1,5]diazocin-8-on,
9-(2-Propyl)-1,2,3,4,5,6-hexahydro-1,5-methano-pyrido[1,2a][1,5]diazocin-8-on,
9-Carbomethoxy-1,2,3,4,5,6-hexahydro-1,5-methano-pyrido[1,2a][1,5]diazocin-8-on,
9-Carboxyaldehyd-1,2,3,4,5,6-hexahydro-1,5-methano-pyrido[1,2a][1,5]diazocin-8-on,
9-(2,6-Difluorophenyl) -1,2,3,4,5,6-hexahydro-1,5-methano-yrido[1,2a][1,5]diazocin-8-on,
9-Phenyl-1,2,3,4,5,6-hexahydro-1,5-methano-pyrido[1,2a][1,5]diazocin-8-on,
9-(2-Fluorophenyl)-1,2,3,4,5,6-hexahydro-1,5-methano-pyrido[1,2a][1,5]diazocin-8-on,
9-(4-Fluorophenyl)-1,2,3,4,5,6-hexahydro-1,5-methano-pyrido[1,2a][1,5]diazocin-8-on,
9-(3-Fluorophenyl)-1,2,3,4,5,6-hexahydro-1,5-methano-pyrido[1,2a][1,5]diazocin-8-on,
9-(3,5-Difluorophenyl)-1,2,3,4,5,6-hexahydro-1,5-methano-yrido[1,2a][1,5]diazocin-8-on,
9-(2,4-Difluorophenyl)-1,2,3,4,5,6-hexahydro-1,5-methano-pyrido[1,2a][1,5]diazocin-8-on,
9-(2,5-Difluorophenyl)-1,2,3,4,5,6-hexahydro-1,5-methano-pyrido[1,2a][1,5]diazocin-8-on,
6-Methyl-5-oxo-6,13-diazatetracyclo[9.3.1.0$^{2,10}$.0$^{4,8}$]pentadeca-2(10).3.8-trien,
5-Oxo-6,13-diazatetracyclo[9.3.1.0$^{2,10}$.0$^{4,8}$]pentadeca-2(10).3.8-trien,
6-Oxo-5,7,13-triazatetracyclo[9.3.1.0$^{2,10}$.0$^{4,8}$]pentadeca-2(10).3.8-trien,
4,5-Difluoro-10-aza-tricyclo[6.3.1.0$^{2,7}$]dodeca-2(7),3,5-trien,
5-Fluoro-10-aza-tricyclo[6.3.1.0$^{2,7}$]dodeca-2(7),3,5-trien-4-carbonitril,
4-Ethynyl-5-fluoro-10-aza-tricyclo[6.3.1.0$^{2,7}$]dodeca-2(7),3,5-trien,
5-Ethynyl-10-aza-tricyclo[6.3.1.0$^{2,7}$]dodeca-2(7),3,5-trien-4-carbonitril,
6-Methyl-5-thia-5-dioxa-6,13-diazatetracyclo[9.3.1.0$^{2,10}$.0$^{4,8}$]pentadeca-2(10).3.8-trien,
10-Aza-tricyclo[6.3.1.0$^{2,7}$]dodeca-2(7),3,5-trien,

4-Fluoro-10-aza-tricyclo[6.3.1.0$^{2,7}$]dodeca-2(7),3,5-trien,

4-Methyl-10-aza-tricyclo[6.3.1.0$^{2,7}$]dodeca-2(7),3,5-trien,

4-Trifluoromethyl-10-aza-tricyclo[6.3.1.0$^{2,7}$]dodeca-2(7),3,5-trien,

4-Nitro-10-azatricyclo[6.3.1.0$^{2,7}$]dodeca-2(7),3,5-trien,

7-Methyl-5,7,13-trazatetracyclo[9.3.1.0$^{2,10}$.0$^{4,8}$]pentadeca-2(10),3,5,8-tetraen,

6-Methyl-5,7,13-triazatetracyclo[9.3.1.0$^{2,10}$.0$^{4,8}$]pentadeca-2(10),3,5,8-tetraen,

6,7-Dimethyl-5,7,13-diazatetracyclo[9.3.1.0$^{2,10}$.0$^{4,8}$]pentadeca-2(10),3,5,8-tetraen,

6-Methyl-7-phenyl-5,7,13-triazatetracyclo[9.3.1.0$^{2,10}$.0$^{4,8}$]pentadeca-2(10),3,5,8-tetraen,

6,7-Dimethyl-5,8,14-triazatetracyclo[10.3.1.0$^{2,11}$.0$^{4,9}$]hexadeca-2(11),3,5,7,9-pentaen,

5,8,14-Triazatetracyclo(10.3.1.0$^{2,11}$.0$^{4,9}$]hexadeca-2(11),3,5,7,9-pentaen,

14-Methyl-5,8,14-triazatetracyclo[10.3.1.0$^{2,11}$.0$^{4,9}$]hexadeca-2(11),3,5,7,9-pentaen,

5-Oxa-7,13-diazatetracyclo[9.3.1.0$^{2,10}$.0$^{4,8}$]pentadeca-2(10).3.6.8-tetraen,

6-Methyl-5-oxa-7,13-diazatetracyclo[9.3.1.0$^{2,10}$.0$^{4,8}$]pentadeca-2(10),3,6,8-tetraen,

4-Chloro-10-azatricyclo[6.3.1.0$^{2,7}$]dodeca-2(7),3,5-trien,

10-Azatricyclo[6.3.1.0$^{2,7}$]dodeca-2(7),3,5-trien-4-yl-cyanid,

1-(10-Azatricyclo[6.3.1.0$^{2,7}$]dodeca-2(7),3,5-trien-4-yl)-1-ethanon,

10-Azatricyclo[6.3.1.0$^{2,7}$]dodeca-2(7),3,5-trien-4-ol,

7-Methyl-5-oxa-6,13-diazatetracyclo[9.3.1.0$^{2,10}$.0$^{4,8}$]pentadeca-2,4(8),6,9-tetraen,

4,5-Dichloro-azatricyclo[6.3.1.0$^{2,7}$]dodeca-2(7),3,5-trien,

11-Azatricyclo[7.3.1.0$^{2,7}$]trideca-2(7),3,5-trien-5-carbonitril,

1-[11-Azatricyclo[7.3.1.0$^{2,7}$]trideca-2(7),3,5-trien-5-yl]-1-ethanon,

1-[11-Azatricyclo[7.3.1.0$^{2,7}$]trideca-2(7),3,5-trien-5-yl]-1-propanon,

4-Fluoro-11-azatricyclo[7.3.1.0$^{2,7}$]trideca-2(7),3,5-trien-5-carbonitril,

5-Fluoro-11-azatricyclo[7.3.1.0$^{2,7}$]trideca-2(7),3,5-trien-4-carbonitril,

6-Methyl-7-thia-5,14-diazatetracyclo[10.3.1.0$^{2,10}$.0$^{4,8}$]hexadeca-2(10),3,5,8-tetraen,

6-Methyl-5,7,14-triazatetracyclo[10.3.1.0$^{2,10}$.0$^{4,8}$]hexadeca-2(10),3,5,8-tetraen,

6,7-Dimethyl-5,7,14-triazatetracyclo[10.3.1.0$^{2,10}$.0$^{4,8}$]hexadeca-2(10),3,5,8-tetraen,

5,7,14-Triazatetracyclo[10.3.1.0$^{2,10}$.0$^{4,8}$]hexadeca-2(10),3,5,8-tetraen,

5,6-Dimethyl-5,7,14-triazatetracyclo[10.3.1.0$^{2,10}$.0$^{4,8}$]hexadeca-2(10),3,6,8-tetraen,

5-Methyl-5,7,14-triazatetracyclo[10.3.1.0$^{2,10}$.0$^{4,8}$]hexadeca-2(10),3,6,8-tetraen,

6-(Trifluoromethyl)-7-thia-5,14-diazatetracyclo[10.3.1.0$^{2,10}$.0$^{4,8}$]hexadeca-2(10),3,5,8-tetraen,

5,8,15-Triazatetracyclo[11.3.1.0$^{2,11}$.0$^{4,9}$]heptadeca-2(11),3,5,7,9-pentaen,

7-Methyl-5,8,15-triazatetracyclo[11.3.1.0$^{2,11}$.0$^{4,9}$]heptadeca-2(11),3,5,7,9-pentaen,

6-Methyl-5,8,15-triazatetracyclo[11.3.1.0$^{2,11}$.0$^{4,9}$]heptadeca-2(11),3,5,7,9-pentaen,

6,7-Dimethyl-5,8,15-triazatetracyclo[11.3.1.0$^{2,11}$.0$^{4,9}$]heptadeca-2(11),3,5,7,9-pentaen,

7-Oxa-5,14-diazatetracyclo[10.3.1.0$^{2,10}$.0$^{4,8}$]hexadeca-2(10),3,5,8-tetraen,

6-Methyl-7-oxa-5,14-diazatetracyclo[10.3.1.0$^{2,10}$.0$^{4,8}$]hexadeca-2(10),3,5,8-tetraen,

5-Methyl-7-oxa-5,14-diazatetracyclo[10.3.1.0$^{2,10}$.0$^{4,8}$]hexadeca-2(10),3,5,8-tetraen,

6-Methyl-5-oxa-5,14-diazatetracyclo[10.3.1.0$^{2,10}$.0$^{4,8}$]hexadeca-2(10),3,6,8-tetraen,

7-Methyl-5-oxa-5,14-diazatetracyclo[10.3.1.0$^{2,10}$.0$^{4,8}$]hexadeca-2(10),3,6,8-tetraen,

4,5-Difluoro-11-azatricyclo[7.3.1.0$^{2,7}$]trideca-2(7),3,5-trien,

4-Chloro-5-fluoro-11-azatricyclo[7.3.1.0$^{2,7}$]trideca-2(7),3,5-trien,

5-Chloro-4-fluoro-11-azatricyclo[7.3.1.0$^{2,7}$]trideca-2(7),3,5-trien,

4-(1-Ethinyl)-5-fluoro-11-azatricyclo[7.3.1.0$^{2,7}$]trideca-2(7),3,5-trien,

5-(1-Ethinyl)-4-fluoro-11-azatricyclo[7.3.1.0$^{2,7}$]trideca-2(7),3,5-trien,

5,6-Difluoro-11-aza-tricyclo[7.3.1.0$^{2,7}$]trideca-2,4,6-trien,

6-Trifluoromethyl-11-aza-tricyclo[7.3.1.0$^{2,7}$]trideca-2,4,6-trien,

6-Methoxy-11-aza-tricyclo[7.3.1.0$^{2,7}$]trideca-2(7),3,5-trien,

11-aza-tricyclo[7.3.1.0$^{2,7}$]trideca-2(7),3,5-trien-6-ol,

6-Fluoro-11-aza-tricyclo[7.3.1.0$^{2,7}$]trideca-2(7),3,5-trien,

11-aza-tricyclo[7.3.1.0$^{2,7}$]trideca-2(7),3,5-trien-5-ol,

4-Nitro-11-aza-tricyclo[7.3.1.0$^{2,7}$]trideca-2(7),3,5-trien,

5-Nitro-11-aza-tricyclo[7.3.1.0$^{2,7}$]trideca-2(7),3,5-trien,

5-Fluoro-11-aza-thicyclo[7.3.1.0$^{2,7}$]trideca-2(7),3,5-trien,

6-Hydroxy-5-methoxy-11-aza-tricyclo[7.3.1.0$^{2,7}$]trideca-2(7),3,5-trien und ihren

pharmazeutisch akzeptablen Salzen und ihren optischen Isomeren.

4. Eine Kombination wie in Anspruch 3 beansprucht worin der Nikotinrezeptor-Partialagonist ausgewählt wird aus:

9-Bromo-1,2,3,4,5,6-hexahydro-1,5-methanopyrido[1,2-a][1,5]diazocin-8-on,

9-Chloro-5,1,2,3,4,5,6-hexahydro-1,5-methanopyrido[1,2-a][1,5]diazocin-8-on,

9-Fluoro-1,2,3,4,5,6-hexahydro-1,5-methanopyrido[1,2-a][1,5]diazocin-8-on,

9-Acetyl-1,2,3,4,5,6-hexahydro-1,5-methanopyrido[1,2a][1,5]diazocin-8-on,

9-Jodo-1,2,3,4,5,6-hexahydro-1,5-methanopyrido[1,2a][1,5]diazocin-8-on,

9-Cyano-1,2,3,4,5,6-hexahydro-1,5-methanopyrido[1,2a][1,5]diazocin-8-on,

9-Carbomethoxy-1,2,3,4,5,6-hexahydro-1,5-methano-pyrido[1,2a][1,5]diazocin-8-on,

9-Carboxyaldehyd-1,2,3,4,5,6-hexahydro-1,5-methano-pyrido[1,2a][1,5]diazocin-8-on,

9-(2,6-Difluorophenyl) -1,2,3,4,5,6-hexahydro-1,5-methano-yrido[1,2a][1,5]diazocin-8-on,

9-Phenyl-1,2,3,4,5,6-hexahydro-1,5-methanopyrido[1,2a][1,5]diazocin-8-on,

9-(2-Fluorophenyl)-1,2,3,4,5,6-hexahydro-1,5-methano-pyrido[1,2a][1,5]diazocin-8-on,

6-Methyl-5-thia-5-dioxa-6,13-diazatetracyclo[9.3.1.0$^{2,10}$.0$^{4,8}$]pentadeca-2(10),3,8-trien,

4-Fluoro-10-aza-tricyclo[6.3.1.0$^{2,7}$]dodeca-2(7),3,5-trien,

4-Trifluoramethyl-10-aza-tricyclo[6.3.1.0$^{2,7}$]dodeca-2(7),3,5-trien,

4-Nitro-10-azatricyclo[6.3.1.0$^{2,7}$]dodeca-2(7),3,5-trien,

6-Methyl-5,7,13-triazatetracyclo[9.3.1.0$^{2,10}$.0$^{4,8}$]pentadeca-2(10),3,5,8-tetraen,

6,7-Dimethyl-5,8,14-triazatetracyclo[10.3.1.0$^{2,11}$.0$^{4,9}$]hexadeca-2(11),3,5,7,9-pentaen,

5,8,14-Triazatetracyclo[10.3.1.0$^{2,11}$.0$^{4,9}$]hexadeca-2(11),3,5,7,9-pentaen,

5-Oxa-7,13-diazatetracyclo[9.3.1.0.$^{2,10}$.0$^{4,8}$]pentadeca-2(10),3,6,8-tetraen,

6-Methyl-5-oxa-7,13-diazatetracyclo[9.3.1.0$^{2,10}$.0$^{4,8}$]pentadeca-2(10),3,6,8-tetraen, 10-Azatricyclo[6.3.1.0$^{2,7}$]dodeca-2(7),

3,5-trien-4-yl-cyanid,

1-(10-Azatricyclo[6.3.1.0$^{2,7}$]dodeca-2(7),3,5-trien-4-yl)-1-ethanon,

11-Azatricyclo[7.3.1.0$^{2,7}$]trideca-2(7),3,5-trien-5-carbonitril,

1-[11-Azatricyclo[7.3.1.0$^{2,7}$]trideca-2(7),3,5-trien-5-yl]-1-ethanon,

1-[11-Azatricyclo[7.3.1.0$^{2,7}$]trideca-2(7),3,5-trien-5-yl]-1-propanon,

4-Fluoro-11-azatricyclo[7.3.1.0$^{2,7}$]trideca-2(7),3,5-trien-5-carbonitril,

5-Fluoro-11-azatricyclo[7.3.1.0$^{2,7}$]trideca-2(7),3,5-trien-4-carbonitril,

6-Methyl-7-thia-5,14-diazatetracyclo[10.3.1.0$^{2,10}$.0$^{4,8}$]hexadeca-2(10),3,5,8-tetraen,

6-Methyl-5,7,14-triazatetracyclo[10.3.1.0$^{2,10}$.0$^{4,8}$]hexadeca-2(10),3,5,8-tetraen,

6,7-Dimethyl-5,7,14-triazatetracyclo[10.3.1.$^{2,10}$.0$^{4,8}$]hexadeca-2(10),3,5,8-tetraen,

6-Methyl-7-oxa-5,14-diazatetracyclo[10.3.1.0$^{2,10}$.0$^{4,8}$]hexadeca-2(10),3,5,8-tetraen,

6-Methyl-5-oxa-5,14-diazatetracyclo[10.3.1.0$^{2,10}$.0$^{4,8}$]hexadeca-2(10),3,6,8-tetraen,

5,6-Difluoro-11-aza-tricyclo[7.3.1.0$^{2,7}$]trideca-2,4,6-trien,

6-Trifluormethyl-11-aza-tricyclo[7.3.1.0$^{2,7}$]trideca-2,4,6-trien,

6-Methoxy-11-aza-tricyclo[7.3.1.0$^{2,7}$]trideca-2(7),3,5-trien,

6-Fluoro-11-aza-tricyclo[7.3.1.0$^{2,7}$]trideca-2(7),3,5-trien,

11-aza-tricyclo[7.3.1.0$^{2,7}$]trideca-2(7),3,5-trien-5-ol und ihren pharmazeutisch akzeptablen Salzen und ihre optischen Isomeren.

5. Eine pharmazeutische Zubereitung umfassend eine Kombination wie in einem jeden der Ansprüche 1 bis 4 beansprucht und ein pharmazeutisch akzeptabler Trägerstoff.

6. Eine Kombination wie in einem jeden der Ansprüche 1 bis 4 beansprucht zur Verwendung als Arzneimittel.

7. Die Verwendung einer Kombination wie in einem jeden der Ansprüche 1 bis 4 beansprucht zur Herstellung eines Arzneimittels zur Behandlung von akutem, chronischem und/oder neuropathischem Schmerz und Migräne.

8. Die Anwendung von Anspruch 7 worin der Nikotinrezeptor-Partialagonist und der NMDA-Rezeptorantagonist im Wesentlichen gleichzeitig verabreicht werden.

**9.** Die Verwendung einer Kombination wie in einem jeden der Ansprüche 1 bis 4 beansprucht zur Behandlung einer Störung oder eines Zustandes ausgewählt aus der Gruppe bestehend aus Erkrankungen und Zuständen bei denen der Schmerz vorherrschend ist, umschließend akuter Schmerz, chronischer Schmerz, neuropathischer Schmerz und Migräne und umschließend Weichgewebe- und periphere Schädigung wie Trauma, Osteoarthritis, rheumatische Arthritis, Muskel-, Knochenschmerz, besonders nach Traumata, Rückenschmerz, Zahnschmerz, myofaciales Schmerzsyndrom, Kopfschmerz, Schnittverletzungsschmerz und Brandschmerz; innere und Leibschmerzen wie Herzschmerz, Muskelschmerz, Augenschmerz, orofazialer Schmerz, zum Beispiel Zahnschmerz, Unterleibsschmerz, gynäkologischer Schmerz, zum Beispiel Dysmenorrhoe, Wehenschmerz, Schmerz in Zusammenhang mit Nerven- oder Nervenbahnen, so wie Schmerz im Zusammenhang mit peripheren Nervenstörungen, zum Beispiel eingeklemmter Nerv, brachiale Plexus Avulsionen, Amputation, periphere Neuropathien, schmerzhafter Tic, atypischer Rückenschmerz, Nervenfaserbeschädigung, und Arachnoditis; Schmerz im Zusammenhang mit Karzinomen, oft als Krebsschmerz bezeichnet; Schmerzen des zentralen Nervensystems, so wie Schmerz, der auf Verletzung des Rückenmarks oder des Stammhirns zurückgeht; Schmerzen im unteren Rückgrat; Ischias, Kopfschmerz einschließlich Migräne, akute oder chronische Verspannungskopfschmerzen, Cluster Kopfschmerzen, temporomandibularer Schmerz und Kiefernhöhlenschmerz; ankylotische Spondylitis; Gicht; postoperativer Schmerz und Narbenschmerzen.

## Revendications

**1.** Association d'un agoniste partial du récepteur de nicotine ou d'un de ses sels pharmaceutiquement acceptables et d'un antagoniste de NMDA ou d'un de ses sels pharmaceutiquement acceptables.

**2.** Association suivant la revendication 1, dans laquelle l'antagoniste de NMDA est choisi entre des dérivés de 2-pipéridino-1-alcanol, le dextrométhorphane, l'éliprodil et l'ifenprodil, leurs sels pharmaceutiquement actifs et leurs isomères optiques.

**3.** Association suivant la revendication 1, dans laquelle l'agoniste partiel du récepteur de nicotine est choisi entre

9-bromo-1,2,3,4,5,6-hexahydro-1,5-méthanopyrido[1,2a][1,5]-diazocine-8-one ;
9-chloro-1,2,3,4,5,6-hexahydro-1,5-méthanopyrido[1,2a][1,5]-diazocine-8-one ;
9-fluoro-1,2,3,4,5,6-hexahydro-1,5-méthanopyrido[1,2a][1,5]-diazocine-8-one ;
9-éthyl-1,2,3,4,5,6-hexahydro-1,5-méthanopyrido[1,2a][1,5]-diazocine-8-one ;
9-méthyl-1,2,3,4,5,6-hexahydro-1,5-méthanopyrido[1,2a][1,5]-diazocine-8-one ;
9-phényl-1,2,3,4,5,6-hexahydro-1,5-méthanopyrido[1,2a][1,5]-diazocine-8-one ;
9-vinyl-1,2,3,4,5,6-hexahydro-1,5-méthanopyrido[1,2a][1,5]-diazocine-8-one ;
9-bromo-3-méthyl-1,2,3,4,5,6-hexahydro-1,5-méthanopyrido-[1,2a][1,5]-diazocine-8-one ;
3-benzyl-9-bromo-1, 2, 3, 4, 5, 6-hexahydro-1,5-méthanopyrido-[1,2a][1,5]-diazocine-8-one ;
3-benzyl-9-chloro-1,2,3,4,5,6-hexahydro-1,5-méthanopyrido[1,2a][1,5]diazocine-8-one ;
9-acétyl-1,2,3,4,5,6-hexahydro-1,5-méthanopyrido[1,2a][1,5]-diazocine-8-one ;
9-iodo-1,2,3,4,5,6-hexahydro-1,5-méthanopyrido[1,2a][1,5]-diazocine-8-one ;
9-cyano-1,2,3,4,5,6-hexahydro-1,5-méthanopyrido[1,2a][1,5]-diazocine-8-one ;
9-éthynyl-1,2,3,4,5,6-hexahydro-1,5-méthanopyrido[1,2a]-[1,5]-diazocine-8-one ;
9-(2-propényl)-1,2,3,4,5,6-hexahydro-1,5-méthanopyrido[1,2a]-[1,5]diazocine-8-one ;
9-(2-propyl)-1,2,3,4,5,6-hexahydro-1,5-méthanopyrido[1,2a]-[1,5]diazocine-8-one;
9-carbométhoxy-1,2,3,4,5,6-hexahydro-1,5-méthanopyrido[1,2a]-[1,5]diazocine-8-one ;
9-carboxyaldéhyde-1,2,3,4,5,6-hexahydro-1,5-méthanopyrido-[1,2a][1,5]diazocine-8-one ;
9-(2,6-difluorophényl)-1,2,3,4,5,6-hexahydro-1,5-méthanopyrido[1,2a][1,5]diazocine-8-one ;
9-phényl-1,2,3,4,5,6-hexahydro-1,5-méthanopyrido[1,2a][1,5]-diazocine-8-one ;
9-(2-fluorophényl)-1,2,3,4,5,6-hexahydro-1,5-méthanopyrido-[1,2a][1,5]diazocine-8-one ;
9-(4-fluorophényl)-1,2,3,4,5,6-hexahydro-1,5-méthanopyrido-[1,2a][1,5]diazocine-8-one ;
9-(3-fluorophényl)-1,2,3,4,5,6-hexahydro-1,5-méthanopyrido-[1,2a][1,5]diazocine-8-one ;
9-(3,5-difluorophényl)-1,2,3,4,5,6-hexahydro-1, 5-méthanopyrido[1,2a][1,5]diazocine-8-one :
9-(2,4-difluorophényl)-1,2,3,4,5,6-hexahydro-1,5-méthanopyrido[1,2a][1,5]diazocine-8-one ;
9-(2,5-difluorophényl)-1,2,3,4,5,6-hexahydro-1,5-méthanopyrido[1,2a][1,5]diazocine-8-one ;
6-méthyl-5-oxo-6,13-diazatétracyclo[9.3 . 1.0$^{2,10}$.0$^{4,8}$]pentadéca-2(10),3,8-triène ;
5-oxo-6,13-diazatétracyclo[9.3.1.0$^{2,10}$.0$^{4,8}$]pentadéca-2(10),3,8-triène ;
6-oxo-5,7,13-triazatétracyclo[9.3.1.0$^{2,10}$.0$^{4,8}$]pentadéca-2(10),3,8-triène ;
4,5-difluoro-10-azatricyclo[6.3.1.0$^{2,7}$]dodéca-2(7),3,5-triène ;

5-fluoro-10-azatricyclo[6.3.1.0$^{2,7}$]dodéca-2 (7), 3,5-triène-4-carbonitrile ;

4-éthynyl-5-fluoro-10-azatricyclo[6.3.1.0$^{2,7}$] dodéca-2(7),3,5-triène ;

5-éthynyl-10-azatricyclo[6.3.1.0$^{2,7}$]dodéca-2 (7), 3, 5-triène-4-carbonitrile ;

6-méthyl-5-thia-5-dioxa-6,13-diazatétracyclo [9.3.1.0$^{2,10}$.0$^{4,8}$]-pentadéca-2(10),3,8-triène ;

10-azatricyclo[6.3.1.0$^{2,7}$]dodeca-2(7), 3,5-triène;

4-fluoro-10-azatricyclo[6.3.1.0$^{2,7}$]dodéca-2 (7),3,5-triène;

4-méthyl-10-azatricyclo[6.3.1.0$^{2,7}$]dodéca-2 (7),3,5-triène;

4-trifluorométhyl-10-azatricyclo[6.3.1.0$^{2,7}$] dodéca-2 (7),3,5-triène ;

4-nitro-10-azatricyclo[6.1.0$^{2,7}$]dodéca-2(7), 3,5-triène ;

7-méthyl-5,7,13-triazatétracyclo[9.3.1.0$^{2,10}$. 0$^{4,8}$]pentadéca-2(10),3,5,8-tétraène ;

6-méthyl-5, 7, 13-triazatétracyclo[9.3.1.0$^{2,10}$. 0$^{4,8}$]pentadéca-2(10),3,5,8-tétraène ;

6,7-diméthyl-5,7,13-triazatétracyclo [9.3.1.0$^{2,10}$.0$^{4,8}$]pentadéca-2(10),3,5,8-tétraène ;

6-méthyl-7-phényl-5,7,13-triazatétracyclo [9.3.1.0$^{2,10}$.0$^{4,8}$]-pentadéca-2(10),3,5,8-tétraène ;

6,7-diméthyl-5,8,14-triazatétracyclo [10.3.1.0$^{2,11}$.0$^{4,9}$]-pentadéca-2(11), 3,5,7,9-tétraène ;

5,8,14-triazatétracyclo[10.3.1.0$^{2,11}$.0$^{4,9}$] hexadéca-2(11),3,5,7,9-pentaène ;

14-méthyl-5,8,14-triazatétracyclo [10.3.1.0$^{2,11}$.0$^{4,9}$]hexadéca-2(11), 3,5,7,9-pentaène ;

5-oxa-7, 13-triazatétracyclo[9.3.1.0$^{2,10}$.0$^{4,8}$] pentadéca-2(10),3,6,8-tétraène ;

6-méthyl-5-oxa-7,13-triazatétracyclo [9.3.1.0$^{2,10}$, 0$^{4,8}$]pentadéca-2(10),3,6,8-tétraène;

4-chloro-10-azatricyclo[6.3.1.0$^{2,7}$]dodéca-2 (7),3,5-triène;

cyanure de 10-azatricyclo[6.3.1.0$^{2,7}$]dodéca-2(7),3,5-triène-4-yle ;

1- (10-azatricyclo[6.3.1.0$^{2,7}$]dodéca-2 (7), 3,5-triène-4-yl) -1-éthanone ;

10-azatricyclo[6.3.1.0$^{2,7}$]dodéca-2(7), 3,5-triène-4-ol;

7-méthyl-5-oxa-6,13-diazatétracyclo [9.3.1.0$^{2,10}$, 0, $^{4,8}$]pentadéca-2,4(8),6,9-tétraène ;

4,5-dichloro-10-azatricyclo[6.3.1.0$^{2,7}$]dodéca-2(7),3,5-triène;

11-azatricyclo[7,3.1.0$^{2,7}$]tridéca-2(7),3,5-triène-5-carbonitrile ;

1-[11-azatricyclo[7.3.1.0,$^{2,7}$]tridéca-2 (7), 3,5-triène-5-yl]-1-éthanone ;

1-[11-azatricyclo[7.3.1. 0$^{2,7}$]tridéca-2(7), 3,5-triène-5-yl]-1-propanone ;

4-fluoro-11-azatricyclo[7.3.1.0$^{2,7}$]tridéca-2

(7),3,5-triène-5-carbonitrile ;

5-fluoro-11-azatricyclo[7.3.1. 0$^{2,7}$]tridéca-2 (7), 3,5-triène-4-carbonitrile ;

6-méthyl-7-thia-5,14-diazatétracyclo [10.3.1.0$^{2,10}$.0$^{4,8}$]hexadéca-2(10),3,5,8-tétraène ;

6-méthyl-5,7,14-triazatétracyclo[10.3.1.0$^{2,10}$. 0$^{4,8}$]hexadéca-2(10),3,5,8-tétraène ;

6,7-diméthyl-5,7,14-triazatétracyclo [10.3.1.0$^{2,10}$.0$^{4,8}$]hexadéca-2(10),3,5,8-tétraène ;

5,7,14-triazatétracyclo[10.3.1.0$^{2,10}$.0$^{4,8}$] hexadéca-2 (10), 3, 5, 8-tétraène ;

5,6-diméthyl-5,7,14-triazatétracyclo [10.3.1.0$^{2,10}$.0$^{4,8}$]hexadéca-2(10),3,6,8-tétraène ;

5-méthyl-5,7,14-triazatétracyclo[10.3.1.0$^{2,10}$. 0$^{4,8}$]hexadéca-2(10),3,6,8-tétraène ;

6-(trifluorométhyl)-7-thia-5,14-diazatétracyclo-[10.3.1.0$^{2,10}$.0$^{4,8}$]hexadéca-2(10),3,5,8-tétraène;

5,8,15-triazatétracyclo[11.3.1.0$^{2,11}$.0$^{4,9}$]heptadéca-2(11),3,5,7,9-pentaène ;

7-méthyl-5, 8,15-triazatétracyclo[11.3.1.0$^{2,11}$. 0$^{4,9}$]heptadéca-2(11),3,5,7,9-pentaène ;

6-méthyl-5,8,15-triazatétracyclo[11.3.1.0$^{2,11}$. 0$^{4,9}$]heptadéca-2(11),3,5,7,9-pentaène ;

6,7-diméthyl-5,8,15-triazatétracyclo [11.3.1.0$^{2,11}$.0$^{4,9}$]heptadéca-2(11), 3,5,7,9-pentaène ;

7-oxa-5,14-diazatétracyclo-[10.3.1.0$^{2,10}$.0$^{4,8}$] hexadéca-2(10),3,5,8-tétraène ;

6-méthyl-7-oxa-5,14-diazatétracyclo [10.3.1.0$^{2,10}$.0$^{4,8}$]hexadéca-2(10),3,5,8-tétraéne ;

5-méthyl-7-oxa-6,14-diazatétracyclo [10.3.1.0$^{2,10}$.0$^{4,8}$]hexadéca-2(10),3,5,8-tétraène ;

6-méthyl-5-oxa-7,14-diazatétracyclo [10.3.1.0$^{2,10}$.0$^{4,8}$]hexadéca-2(10),3,6,8-tétraène ;

7-méthyl-5-oxa-6,14-diazatétracyclo [10.3.1.0$^{2,10}$.0$^{4,8}$]hexadéca-2(10),3,6,8-tétraène ;

4,5-difluoro-11-azatricyclo[7.3.1.0$^{2,7}$]tridéca-2 (7),3,5-triène ;

9-chloro-5-fluoro-11-azatricyclo[7.3.1.0$^{2,7}$]tridéca-2(7), 3,5-triène ;

5-chloro-4-fluoro-11-azatricyclo[7.3.1.0$^{2,7}$]tridéca-2 (7), 3, 5-triène ;

4-(1-éthynyl)-5-fluoro-11-azatricyclo[7.3 . 1.0$^{2,7}$]tridéca-2(7),3,5-triène ;

5-(1-éthynyl)-4-fluoro-11-azatricyclo [7.3.1.0$^{2,7}$]tridéca-2(7),3,5-triène ;

5,6-difluoro-11-azatricyclo[7.3.1.0$^{2,7}$]tridéca-2,4,6-triène;

6-trifluorométhyl-11-azatricyclo[7.3.1.0$^{2,7}$]-tridéca-2,4,6-triène ;

6-méthoxy-11-azatricyclo[7.3.1.0$^{2,7}$]tridéca-2 (7),3,5-triène ;

11-azatricyclo[7.3.1.0,$^{2,7}$]tridéca-2(7),3,5-triène-6-ol ;

6-fluoro-11-azatricyclo[7.3.1.0$^{2,7}$]tridéca-2 (7),3,5-triène ;

11-azatricyclo[7.3.1.0$^{2,7}$]tridéca-2(7),3,5-triène-5-ol ;

4-nitro-11-azatricyclo[7.3.1.0$^{2,7}$]tridéca-2(7),3,5-triène ;

5-nitro-11-azatricyclo[7.3.1.0$^{2,7}$]tridéca-2(7),3,5-triène ;

5-fluoro-11-azatricyclo[7.3.1.0$^{2,7}$]tridéca-2(7),3,5-triène ;

6-hydroxy-5-méthoxy-11-azatricyclo[7.3.1.0$^{2,7}$]tridéca-2(7), 3,5-triène ; et

leurs sels pharmaceutiquement acceptables et leurs isomères optiques.

4. Association suivant la revendication 3, dans laquelle l'agoniste partiel du récepteur de nicotine est choisi entre

9-bromo-1, 2, 3, 4, 5, 6-hexahydro-1, 5-méthanopyrido[1, 2a][1, 5]-diazocine-8-one ;

9-chloro-1,2,3,4,5,6-hexahydro-1,5-méthanopyrido[1,2a][1,5]-diazocine-8-one ;

9-fluoro-1,2,3,4,5,6-hexahydro-1,5-méthanopyrido[1,2a][1,5]-diazocine-8-one ;

9-acétyl-1,2,3,4,5,6-hexahydro-1,5-méthanopyrido[1,2a][1,5]-diazocine-8-one ;

9-iodo-1,2,3,4,5,6-hexahydro-1,5-méthanopyrido[1,2a][1,5]-diazocine-8-one ;

9-cyano-1,2,3,4,5,6-hexahydro-1,5-méthanopyrido[1,2a][1,5]-diazocine-8-one ;

9-carbométhoxy-1,2,3,4,5,6-hexahydro-1,5-méthanopyrido[1,2a]-[1,5]-diazocine-8-one ;

9-carboxyaldéhyde-1,2,3,4,5,6-hexahydro-1,5-méthanopyrido-[1,2a][1,5]-diazocine-8-one ;

9-(2,6-difluorophényl)-1,2,3,4,5,6-hexahydro-1,5-méthanopyrido[1,2a][1,5]diazocine-8-one ;

9-phényl-1,2,3,4,5,6-hexahydro-1,5-méthanopyrido[1,2a][1,5]-diazocine-8-one ;

9-(2-flurophényl)-1,2,3,4,5,6-hexahydro-1,5-méthanopyrido-[1,2a][1,5]-diazocine-8-one ;

6-méthyl-5-thia-5-dioxa-6,13-diazatétracyclo-[9.3.1.0$^{2,10}$.0$^{4,8}$]-pentadéca-2(10),3,8-triène ;

4-fluoro-10-azatricyclo[6.3.1.0$^{2,7}$]dodéca-2(7),3,5-triène ;

4-trifluorométhyl-10-azatricyclo[6.3.1.0$^{2,7}$]dodéca-2(7),3,5-triène ;

4-nitro-10-azatricyclo[6.3.1.0$^{2,7}$]dodéca-2(7),3,5-triène ;

6-méthyl-5,7,13-triazatétracyclo[9.3.1.0$^{2,10}$.0$^{4,8}$]pentadéca-2(10),3,5,8-tétraène ;

6,7-diméthyl-5,8,14-triazatétracyclo[10.3.1.0$^{2,11}$.0$^{4,9}$]hexadéca-2(11),3,5,7,9-pentaène;

5,8,14-triazatétracyclo[10.3.1.0$^{2,11}$.0$^{4,9}$]hexadéca-2(11),3,5,7,9-pentaène ;

5-oxa-7,13-diazatétracyclo[9.3.1.0$^{2,10}$.0$^{4,8}$]pentadéca-2(10),3,6,8-tétraène ;

6-méthyl-5-oxa-7,13-diazatétracyclo[9.3.1.0$^{2,10}$.0$^{4,8}$]pentadéca-2(10),3,6,8-tétraène ;

cyanure de 10-azatricyclo(6.3.1.0$^{2,7}$]dodéca-2(7),3,5-triène-4-yle ;

1-(10-azatricyclo[6.3.1.0$^{2,7}$]dodéca-2(7),3,5-triène-4-yl)-1-éthanone ;

11-azatricyclo[7.3.1.0$^{2,7}$]tridéca-2(7),3,5-triène-5-carbonitrile ;

1-[11-azatricyclo[7.3.1.0$^{2,7}$]tridéca-2(7),3,5-triène-5-yl]-1-éthanone ;

1-[11-azatricyclo[7.3.1.0$^{2,7}$]tridéca-2(7),3,5-triène-5-yl]-1-propanone ;

4-fluoro-11-azatricyclo[7.3.1.0$^{2,7}$]tridéca-2(7),3,5-triène-5-carbonitrile ;

5-fluoro-11-azatricyclo[7.3.1.0$^{2,7}$]tridéca-2(7),3,5-triène-4-carbonitrile ;

6-méthyl-7-thia-5,14-diazatétracyclo[10.3.1.0$^{2,10}$.0$^{4,8}$]hexadéca-2(10),3,5,8-tétraène;

6-méthyl-5,7,14-triazatétracyclo[10.3.1.0$^{2,10}$.0$^{4,8}$]hexadéca-2(10),3,5,8-tétraène ;

6,7-diméthyl-5,7,14-triazatétracyclo[10.3.1.0$^{2,10}$.0$^{4,8}$]hexadéca-2(10),3,5,8-tétraène ;

6-méthyl-7-oxa-5,14-diazatétracyclo[10.3.1.0$^{2,10}$.0$^{4,8}$]hexadéca-2(10),3,5,8-tétraène ;

6-méthyl-5-oxa-7,14-diazatétracyclo[10.3.1.0$^{2,10}$.0$^{4,8}$]hexadéca-2(10),3,6,8-tétraène ;

5, 6-difluoro-11-azatricyclo[7.3.1.0$^{2,7}$]tridéca-2,4,6-triène;

6-trifluorométhyl-11-azatricyclo[7.3.1.0$^{2,7}$]tridéca-2,4,6-triène ;

6-méthoxy-11-azatricyclo[7.3.1.0$^{2,7}$]tridéca-2(7),3,5-triène ;

6-fluoro-11-azatricyclo[7.3.1.0$^{2,7}$]tridéca-2(7),3,5-triène ; et

11-azatricyclo[7.3.1.0$^{2,7}$]tridéca-2(7),3,5-triène-5-ol;

et leurs sels pharmaceutiquement acceptables ainsi que leurs isomères optiques.

5. Composition pharmaceutique comprenant une association suivant l'une quelconque des revendications 1 à 4 et un support pharmaceutiquement acceptable.

6. Association suivant l'une quelconque des revendications 1 à 4, destinée à être utilisée comme médicament.

7. Utilisation d'une association suivant l'une quelconque des revendications 1 à 4 pour la production d'un médicament destiné au traitement de la douleur aiguë, chronique et/ou neuropathique et de la migraine.

8. Utilisation suivant la revendication 7, dans laquelle l'agoniste partiel du récepteur de nicotine et l'antagoniste du récepteur de NMDA sont administrés pratiquement simultanément.

9. Utilisation d'une association suivant l'une quelconque des revendications 1 à 4 pour la production d'un médicament destiné au traitement d'un trouble ou d'une affection choisi dans le groupe consistant en des maladies et affections dans lesquelles la douleur prédomine, comprenant la douleur aiguë, la douleur chronique, la douleur neuropathique et la migraine, et comprenant une altération des tissus mous et une altération périphérique, telle qu'un traumatisme aigu, l'ostéoarthrite, la polyarthrite rhumatoïde, la douleur musculosquelettique, en particulier après un traumatisme, la douleur rachidienne, la douleur dentaire, des syndromes de douleur myofaciale, les céphalées, la douleur provoquée par une épisiotomie et des brûlures ; la douleur profonde et la douleur viscérale, telle qu'une douleur cardiaque, une douleur musculaire, une douleur oculaire, une douleur buccofaciale, par exemple l'odontalgie, une douleur abdominale, une douleur gynécologique, par exemple la dysménorrhée et la douleur de l'accouchement ; la douleur associée à une lésion des nerfs et des racines, telle que la douleur associée à des troubles des nerfs périphériques, par exemple l'englobement de nerfs et des avulsions du plexus brachial, une amputation, les neuropathies périphériques, un tic douloureux, une douleur faciale atypique, une altération d'une racine nerveuse et l'arachnoïdite ; la douleur associée à un carcinome, désignée souvent sous le nom de douleur du cancer ; une douleur du système nerveux central, telle que la douleur due à une altération de la moelle épinière ou du tronc cérébral ; une douleur dans le bas du dos ; la sciatique ; des céphalées, comprenant la migraine, des céphalées aiguës ou chroniques dues à la tension, les céphalées vasculaires de Horton, la douleur temporomandibulaire et la douleur du sinus maxillaire ; la spondylite ankylosante, la goutte ; une douleur postopératoire ; et la douleur provoquée par une cicatrice.